# EUROPEAN PATENT SPECIFICATION

(11) **EP 3 277 328 B1**
(45) Date of publication and mention of the grant of the patent: **07.06.2023**
(21) Application number: 16718751.7
(22) Date of filing: 31.03.2016
(51) Int. Cl.: A61L 27/04, A61L 27/06, A61L 27/52, A61L 27/54, A61L 31/02, A61L 31/16, A61L 24/02, A61L 27/44, A61L 31/12, A61L 24/00, C08L 67/04

(54) **FERROMAGNETIC PARTICLES BOUND TO POLYMERIC IMPLANTS**
AN POLYMERIMPLANTATE GEBUNDENE FERROMAGNETISCHE TEILCHEN
PARTICULES FERROMAGNÉTIQUES LIÉES À DES IMPLANTS POLYMÈRES

(30) Priority: 01.04.2015 US 201562141720 P
(43) Date of publication of application: 07.02.2018
(73) Proprietor: Yale University, New Haven, CT 06510 (US)
(72) Inventor: FAHMY, Tarek, New Haven, CT 06511 (US); SINUSAS, Albert, Guilford, CT 06437 (US); LEE, Jung, Seok, New Haven, CT 06511 (US); KIM, Dongin, College Station, TX 79845 (US)
(74) Representative: Potter Clarkson
(86) International application number: PCT/US2016/025328
(87) International publication number: WO 2016/161148

(56) References cited:
- WO-A2-2008/034050
- WO-A2-2014/021954
- SHOUHENG SUN: "Recent advances in chemical synthesis, self-assembly and applications of FePt nanoparticles", ADVANCED MATERIALS, VCH PUBLISHERS, vol. 18, 1 January 2006 (2006-01-01), pages 393-403, XP008165201, ISSN: 0935-9648, DOI: 10.1002/ADMA.200501464

## Description

### CROSS REFERENCE TO RELATED APPLICATION

This application claims benefit of and priority to U.S. Provisional Application No. 62/141,720, filed April 1, 2015.

### STATEMENT REGARDING FEDERALLY SPONSORED RESEARCH

This invention was made with government support under T32 grant Award Number 5T32HL098069 awarded by the National Institutes of Health. The government has certain rights in the invention.

### FIELD OF THE INVENTION

The field of the invention is generally related to localized cell attraction, capture, and retention *in vivo,* and more particularly to compositions and methods for attracting, capturing, and retaining target cells to a site where tissue repair and/or regeneration is needed, using a minimally invasive procedure.

### BACKGROUND OF THE INVENTION

Tissue engineering is a rapidly expanding interdisciplinary field involving biomaterials science, cell biology, cell-material interactions and surface characterization. Research in this field aims to restore, preserve, or enhance tissue functions. It also aims to replace diseased or damaged organs, or tissues that are defective or have been lost as a result of accidents or disease. Tissue engineering typically involves four key components: (a) selected and isolated cells, (b) biomaterial scaffolds which may be natural or synthetic, to provide a platform for cell function, adhesion and transplantation, optionally (c) signaling molecules such as proteins and growth factors deriving the cellular functions of interest, and optionally (d) bioreactors that support a biologically active environment for cell expansion and differentiation such as cell culture. Tissues or organs can be potentially developed via a number of approaches. The most common approach involves isolation of tissue-specific cells from the patient's small tissue biopsy and harvested *in vitro.* The isolated cells are then expanded and seeded into three-dimensional scaffold that mimic the natural extracellular matrices (ECM) of the targeted tissues. The key functions of these scaffolds are to (a) deliver the seeded cells to the desired site in the patient's body, (b) encourage cell-biomaterial interactions, (c) promote cell adhesion, (d) permit adequate transport of gases, nutrients and growth factors to ensure cell survival, proliferation, and differentiation, (e) confer a negligible inflammation extent or toxicity in vivo, and (f) control the structure and function of the engineered tissue. 1 The cell-loaded scaffolds are subsequently transplanted into the patient either through direct injection with the aid of a needle or other minimally invasive delivery technique, or through implantation of the fabricated tissue at the desired site in the patient's body using surgery. WO 2008/034050 A2 describes a polymeric implant such as a stent comprising magnetic particles like Fe/Pt particles coated with silica.

It would be highly desirable to be able to provide means for tissue engineering without having to first culture and then seed cells, using an open surgical procedure. Previous efforts to use an injectable hydrogel material having cells dispersed therein, such as that described by U.S. Patent No. 5,709,854, were unsuccessful except in the case of chondrocytes due to difficulties in obtaining a sufficiently high density in the absence of vascularization and being unable to subsequently inject cells into the scaffold and obtain a uniform high density dispersion.

Therefore, it is an object of the invention to provide materials for enhanced retention of cells on injectable tissue engineering scaffolds.

It is a further object of the invention to provide materials for enhancing tissue repair, particularly at sites of injury.

It is also an object of the invention to provide materials for the tracking of tissue repair, cell accumulation to the tissue engineering scaffold, and noninvasive or invasive evaluation of the engineered tissue.

### SUMMARY OF THE INVENTION

It has been discovered that magnetic particles such as iron or iron-platinum magnetic particles can be dispersed and/or coated into or onto, or directly linked to, a hydrogel polymer, bone cement, or polymer scaffold and magnetized. The magnetized materials are used to attract, capture, and/or retain magnetically labeled cells on the polymer *in vivo.* The magnetic devices are particularly useful for capturing and retaining cells exposed to the stresses and forces of biological fluid flow. They can also be used to increase tissue integration at the site of implantation of a prosthesis such as a metal surface hip or knee prosthetic, or decrease bone erosion surrounding a metal bone screw, pin or plate.

The invention is as defined in claim 1. Previous versions were not annealed (i.e., not heated to create the L1₀ crystalline phase needed to hold a magnetic moment). As such they were superparamagnetic and thus displayed no hysteresis in the magnetization curve (i.e., not ferromagnetic). Annealing the Fe:Pt is very important for introducing a crystal structure LI0 interior crystalline phase. Annealing takes place at temperatures over 600°C. The introduction of the L1₀ interior crystalline phase changes the material from a paramagnetic material to a ferromagnetic material, such that it becomes a permanent magnet when exposed to a magnetic field. In a preferred embodiment, the particles are annealed at 700°C for 30 min. This creates the magnetization.

In certain applications, it may be advantageous to have a device that can be permanently magnetized. For example, this may be useful in the case of repeated administration of cells, or in the case that access to a magnetic field is difficult. Further, there are advantages to avoiding magnetization *in situ,* since magnetization *in situ* may cause the device to move, which may cause physiological problems.

Particle disintegration may be minimized by coating the Fe/Pt with Silica then heating to prevent particle disintegration. The Fe/Pt molar ratio for creation of the crystal phase is important and should be in a range where Fe/Pt particles are in an L1₀ crystalline phase. The skilled person will know the molar ratio that is required to form this crystalline phase, but a preferred range, expressed as an average compositional molar ratio of Fe to Pt, is in the range 40:60 +/- 10:10 mol%, and preferably +/- 5:5. The magnetic force as a whole can be measured with a "Super Conducting Quantum Interference Device", which is a sensitive magnetometer. The overall magnetic force is preferably in the range from 0.01 to 2.0 Tesla, preferably 0.01 to 1.5 Tesla. Further preferred upper limits are 1.0 or 0.5 T. The skilled person will understand that the magnetic force should be tailored to the application. For example, a small magnetic force would be adequate, if there are a small number of cells, and if those cells need to be attracted for only a short amount of time. The upper limit of the magnetic force is important as at higher levels, the magnetic force could be physiologically detrimental.

The particles can be coupled to polymers which are crosslinked to form an injectable scaffold or formed into a mesh, sutures, or bone cements. The particles can be encapsulated with and/or are functionalized with reactive groups, imaging or contrast agents such as iodine, and/or therapeutic or prophylactic agents. In preferred embodiments, the polymer forming the scaffolds is a polyester, more preferably a polyhydroxy acid polymer, most preferably poly(lactic acid) (PLA), poly(glycolic acid) (PGA), poly(lactide-co-glycolide) (PLGA), or poly-L-lactide (PLLA) or a hydrogel forming polymer such as alginate or a pluronic. The magnetic particles are typically between 1% and 30% of the polymer by weight, inclusive, for Fe/Pt particles of greater than 50% Fe per Fe/Pt particle, and between 5% and 30% for Fe/Pt particles of less than 50% Fe per Fe/Pt particle of the polymer by weight.

Cells are magnetized by binding to, or incorporation of, particles such as iron oxide particles or other metal particles binding to Fe/Pt which has been magnetized. There are a number of commercial reagents containing iron oxide particles coupled to antibodies which specifically bind to ligands on the cell surfaces. Iron oxide particles can also be incorporated into phagocytic cells by culturing in cell media containing the iron oxide particles. Commercially available systems can be used for isolation of the cells containing or having bound thereto iron oxide particles.

The examples demonstrate biodistribution of Fe/Pt particles encapsulated in polymer formulation and alone. The particles were directly decorated with an infrared dye for visualization. Toxicology studies were also performed with different doses of Fe/Pt particles. The impact of various parameters on cell capture; including flowrate, initial cell concentration, and density of cells, was also determined. The results show that 5-30% by weight of Fe/Pt particles to polymer is the working range to produce a scaffold coating sufficiently magnetic to capture and/or retain magnetic cells on or adjacent to the scaffold for at least 1, 2, 3, 4, 5, 6, 7, or more days, weeks, or months under biological flow *in vivo.*

Methods of treatment using the disclosed magnetized scaffolds are also disclosed but not claimed. For example, a method of treating an orthopedic injury can include implanting a magnetized bone cement into a defect, either having magnetic cells dispersed therein or subsequently administering magnetic cells, such as mesenchymal stem cells or osteoblasts. In other embodiments, the Fe/Pt particles (referred herein unless otherwise specified as "iron particles") are bound to biodegradable mesh such as PLGA woven or non-woven mesh placed onto a non-healing diabetic ulcer, then applying magnetic cells such as endothelial, epithelial or mesenchymal stem cells to the mesh. In other embodiments, the iron particles are bound to alginate, which is dissolved in a pharmaceutically acceptable carrier, cells such as myocytes dispersed therein, calcium added, and the suspension injected into damaged heart muscle. In another embodiment, the cells are chondrocytes or fibroblasts, and the suspension is injected adjacent to injured cartilage, tendon or ligament. Some embodiments further include administering to the subject therapeutic, prophylactic or diagnostic agent with the magnetic cells to enhance or increase repair or visualization of injury.

### BRIEF DESCRIPTION OF THE DRAWINGS

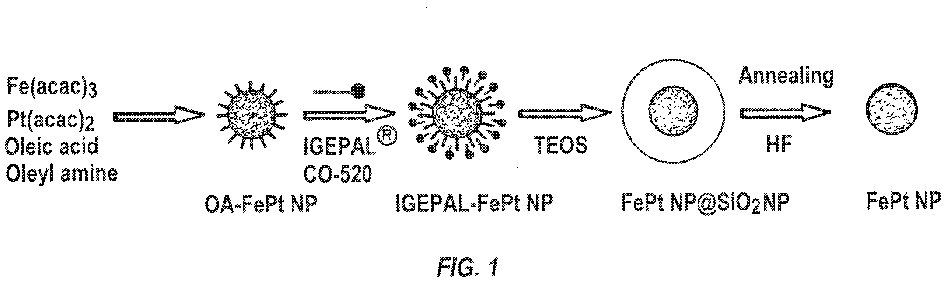

Figure 1 is a flow diagram showing the fabrication of an exemplary iron/platinum (Fe/Pt) particle.

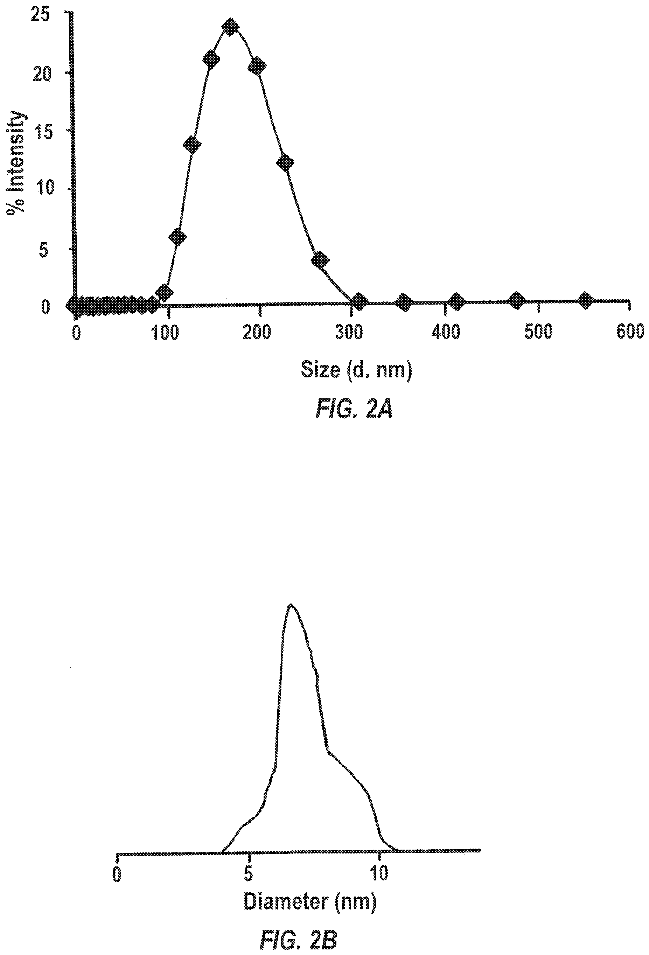

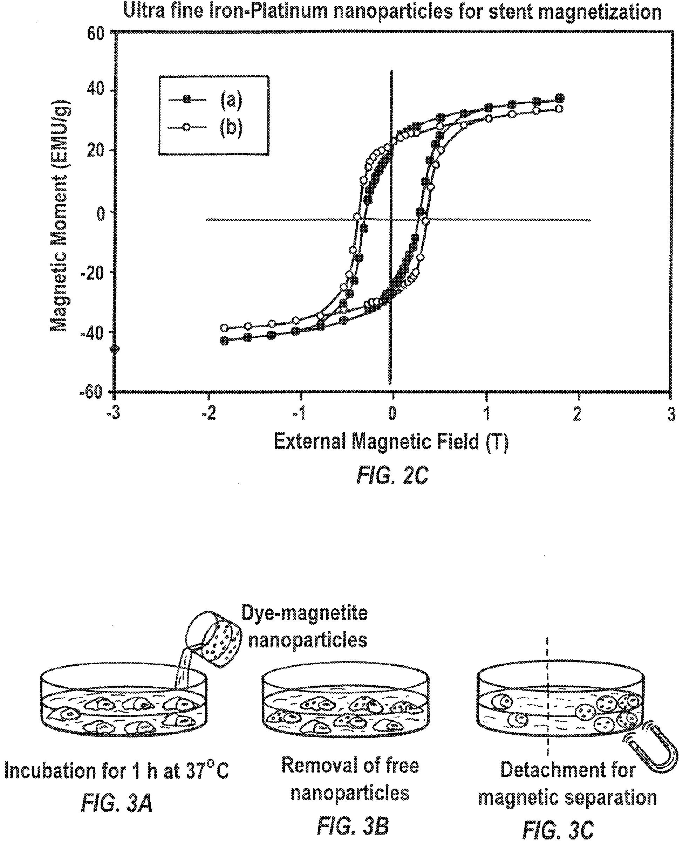

Figures 2A-B are plots illustrating the size distribution of the Fe/Pt particles. Figure 2C is a hysteresis loop plot showing the magnetic moment (EMU/g) verse the external magnetic field of ((a) Fe/Pt particles annealed in N₂ at 600 °C for 30 min, and (b) Fe/Pt particles made in forming gas (N₂ 93% and H₂ 7%) at 600 °C for 30 min.
Figures 3A-3C exemplify the method of adding iron oxide particles to cells in culture (3A), which are taken up by the cells by phagocytosis or bound by a ligand such as an antibody (3B), and then isolated using an external magnet (3C).

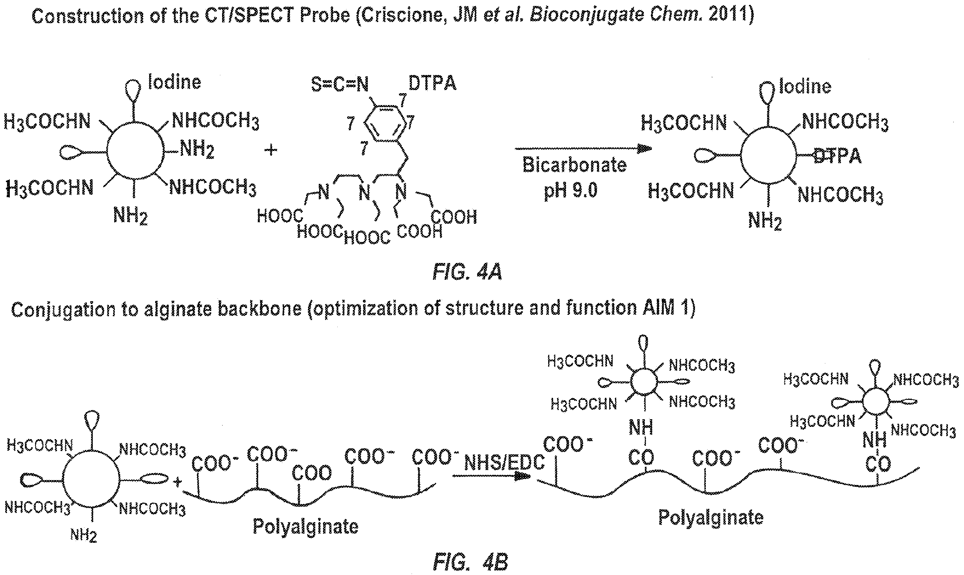

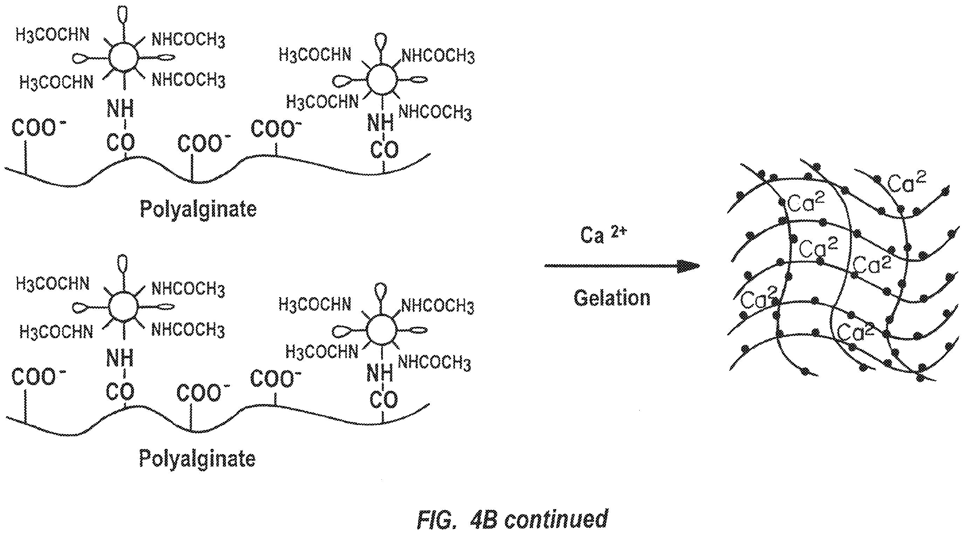

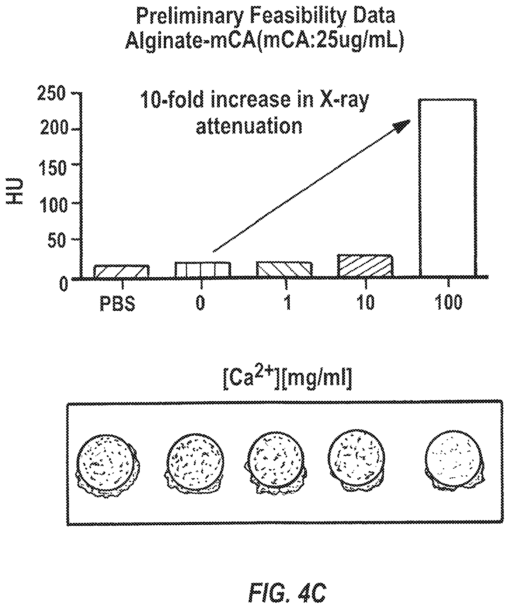

Figure 4A is a diagram showing particles functionalized with DTPA and iodine. Figure 4B is a diagram showing conjugation of functionalized particles to polyalginate which can be magnetized and crosslinked with Calcium (Ca2+) to form magnetic alginate hydrogel. Figure 4C is a bar graph showing the x-ray attenuation (Hounsfield unit (HU)) of iodine-functionalized-magnetic particle-conjugated polyalginate in the presence of increasing amounts of crosslinker (Ca2+).

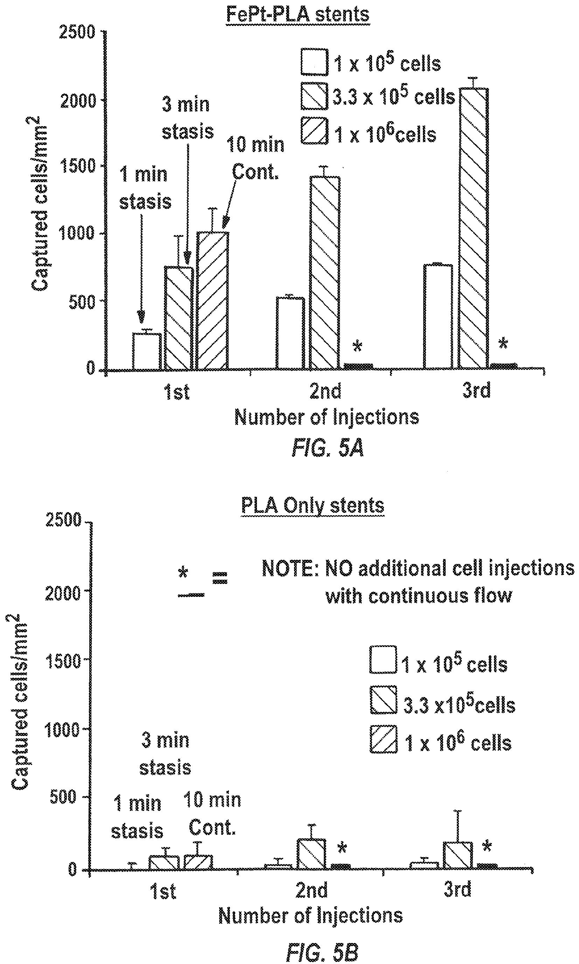

Figure 5A is a graph showing number of injections (x-axis) versus number of captured cells (magnetized CD34+ stem cells) per mm², when the magnetized cells were flowed on magnetized FePt-PLA stents. Figure 5B is a graph showing number of injections (x-axis) versus number of captured cells (magnetized CD34+ stem cells) per mm², when the magnetized cells were flowed on control PLA-only stents.

### DETAILED DESCRIPTION OF THE INVENTION

### I. Magnetic Particles, Polymeric Materials and Cells

A system has been developed for selective adherence of cells to polymeric implants in a body.

The polymeric materials have directly coupled thereto, or are coated with, Fe/Pt particles having a crystalline structure allowing them to be magnetized upon exposure to an external magnetic source following implantation. The Fe/Pt particles display hysteresis in the magnetization curve (i.e., are ferromagnetic).

The cells are adhered to the scaffolds post implantation by administration of cells having incorporated therein or bound thereto magnetic particles such as iron oxide.

### A. Magnetic Particles

There are two types of magnetic particles: those bound to the scaffold which must be ferromagnetic and those which are incorporated into or onto the cells which bind to the scaffolds. "Magnetizable particles" are particles that are capable of being magnetized when placed in an external magnetic field. Methods of magnetizing particles with an external magnetic field are known in the art. The magnetizing step can occur before, during, or after the magnetic particles are incorporated into the scaffold or cell. The magnetism of the magnetic particles can be permanent or transient. The magnetic particles can be re-magnetized.

### Magnetic Particles for Binding to or Incorporation into Cells

The magnetic particles can be ferromagnetic particles (i.e., iron-containing particles providing electrical conduction or resistance). Suitable ferromagnetic particles include iron-containing magnetic metal oxides (paramagnetic or superparamagnetic), for example, those including iron either as Fe(II), Fe(III), or a mixture of Fe(II)/Fe(III). Non-limiting examples of such oxides include FeO, γ-Fe₂O₃ (maghemite), and Fe₃O₄ (magnetite). The magnetic particles can also be a mixed metal oxide of the type M1ₓM2₃₋ₓO₄, wherein M1 represents a divalent metal ion and M2 represents a trivalent metal ion. For example, the magnetic particles may be magnetic ferrites of the formula M1Fe₂O₄, wherein M1 represents a divalent ion selected from Mn, Co, Ni, Cu, Zn, or Ba, pure or in admixture with each other or in admixture with ferrous ions. Other metal oxides include aluminium oxide, chromium oxide, copper oxide, manganese oxide, lead oxide, tin oxide, titanium oxide, zinc oxide and zirconium oxide, and suitable metals include Fe, Cr, Ni or magnetic alloys.

The particles can be Co particles (e.g., J. Appl. Phys. 1999, 85, 4325), FePt alloy particles (e.g., Science 2000, 287, 1989), FePd or CoPd particles (e.g., J. Appl. Phys. 2002, 91, 8477), Mn₃O₄ or MnO particles (e.g., Angew. Chem. Int. Ed 2004, 43, 1115), Ni particles (e.g., Adv. Mater. 2005, 17, 429), (Y₁₋ₓGdₓ)₂O₃ particles, wherein x is from 0 to 1, (e.g., Chem. Mater. 2008, 20, 2274).

Commercially available magnetic particles may be used, such as Iron55-nickel45 alloy nanopowder (<100 nm) available from Aldrich, Iron nickel oxide 98% nanopowder Fe₂NiO₄ 20-30 nm available from Aldrich, iron oxide Fe₃O₄ nanopowder >98% 20-30 nm available from Merck, nickel cobalt oxide nanopowder 99% NiO CoO<30 nm available from Aldrich, cobalt (II III) oxide nanopowder 99.8% 20-30 nm available from Merck, nickel(II) oxide nanopowder 99.8% 10-20 nm available from Merck, gadolinium (III) oxide nanopowder 99.9+%<40 nm available from Aldrich, nickel zinc iron oxide nanopowder 99% available from Aldrich, copper zinc iron oxide nanopowder, <80 nm, 98.5% available from Aldrich, copper iron oxide nanopowder 98.5% available from Aldrich, but it is not limited thereto.

The magnetic particles include an alloy such as FePt, FeCo, or CoPt. Specific preferred particles are reviewed in Ho, et al., Acc Chem Res., 44(10): 875-882 (2011) and include magnetite (Fe₃O₄), ferrite MFe₂O₄ (M = Mn, Zn); Au-Fe₃O₄, metallic Fe, FePt alloy, FeCo alloy particles, or a combination thereof.

### Magnetic Particles for Binding to or Coating Onto Scaffolds

The magnetic particles are iron/platinum (FePt) particles. Methods of making magnetic particles are known in the art. See for example, Sun, et al., IEEE Trans. Magn., 37:1239-1243 (2001), which described FePt particles prepared by the reduction of Pt(acac)₂ and the decomposition of Fe(CO)₅. Other methods included addition of Ag, Co to the FePt particles to improve their physical and magnetic properties (Shevchenko, et al., J. Am. Chem. Soc., 124(38):11480-11485 (2002), Kang, et al., Nano Lett., 2(3):1033-1036 (2002)), the formation of FePt particles by the simultaneous reduction of FeCl2 and Pt(acac)₂, and Fe and Pt acetylacetonate (Sun, et al., IEEE Trans. Magn., 37:1239-1243 (2001), Jeyadevan, et al., J. Appl. Phys., 93(10):7574 (2003)). Particle size produced by the above-mentioned methods is generally around 3-4 nm. A method of making two nm diameter FePt particles is described in Elkins, et al., Nano Letters, 3(12):1647-49 (2003).

For example, the synthesis can include simultaneous chemical reduction of Pt(acac)₂ and Fe(acac)₃ by 1,2-hexadecanediol at high temperature (e.g., 250 °C) in solution phase, under standard airless techniques in an argon atmosphere. For example, a molar ratio of about 1:2:10 of Pt(acac)₂:Fe(acac)₃:1,2-hexadecanediol (e.g., 0.5 mmol: 1.0 mmol:5.0 mmol) is mixed. A suitable volume of dioctyl ether is added and mixed while purging with Ar. The mixture is heated to a suitable temperature, for example 100 °C, and maintained for a suitable period of time (e.g., 20 min). During this hold, suitable amounts of oleylamine and oleic acid (e.g., 0.05 mmol (0.17 mL) of oleylamine and 0.05 mmol (0.16 mL) of oleic acid) are injected into the mixture while continuing the Ar purge. After the hold, the mixture is maintained under an Ar blanket and heated further heated (e.g., to about 250 °C) at a suitable rate (e.g., about 7 °C per minute (reflux)), and maintained the temperature for a suitable amount of time (e.g., about 30 min) before cooling down to room temperature under the Ar blanket. Afterward, all handling can be performed open to the atmosphere.

Purification can include mixing the dispersion with ethyl alcohol (EtOH), collecting the precipitate, and discarding the supernatant. The precipitate can be redispersed in hexane and EtOH (e.g., ratio of 2:1). Additional small amount of oleylamine and oleic acid can optionally be added to aid in redispersing the particles. The supernatant of the redispersion can be collected and transferred to a new centrifuge tube, discarding any precipitate that separates. Additional EtOH can be added to this dispersion. The supernatant can be discarded and the remaining dark brown precipitate redispersed in hexane or dried for storage.

The FePt particles can coated with SiO₂ by base-catalyzed silica formation from tetraethylorthosilicate in a water-in-oil microemulsion in order to reduce the thermal aggregation of FePt particles during annealing at high temperature. Such methods are known in the art. See, for example, Lee, Silicon Nanowires, Carbon Nanotubes, and Magnetic Nanocrystals: Synthesis, Properties, and Applications, ProQuest Information and Learning Comp., Ann Arbor, MI (2007). For example, Igepal CO-520 can be mixed with cyclohexane. FePt particles dispersed in cyclohexane can be injected into the cyclohexane/Igepal solution. 30% NH₄OH aqueous solution can be added, followed by the addition of tetraethylorthosilicate (TEOS). The mixture is typically stirred for several days (e.g., 72 h) before adding methanol to collect particles. The particles can be precipitated with excess hexane and collected (e.g., by centrifugation). The particles can be redispersed in ethanol. The FePt/SiO₂ particles can be "washed" using this procedure at least three times to remove excess surfactant.

The FePt/SiO₂ particles are annealed at high temperature, for example, using a tube furnace. The particles can be drop-cast onto a Si wafer, positioned into a quartz tube, and then placed in the tube furnace. Annealing can be carried out by purging the tube and the sample with 7% H₂/93% N₂ flow at 700 °C. After annealing, SiO₂ coating can be removed by treating the particles with 1% hydrofluoric acid (HF) solution.

The magnetic particles are iron platinum (FePt) particles. The Examples discussed below illustrate that FePt particles made by this process have magnetic retention of at least 60 days, which will provide sufficient timing, for example, for the attraction of iron-labeled endothelial cells (EPC) to a magnetized stent post-implantation. Methods of making such magnetic particles are known in the art as described further below including exemplary methods for synthesizing FePt particles.

In some embodiments, the FePt particles have a Fe:Pt molar ratio in the range of about 1:10 to about 10:1. In a preferred embodiment, the FePt particle composition has a Fe:Pt molar ratio of about 1:1. In certain embodiments, the Fe molar percentage of the FePt particle may be as low as 5-10% and sufficient particle magnetization is still achieved In a preferred embodiment, average compositional molar ratio of Fe to Pt, is in the range 40:60 +/- 10: 10 mol%, and preferably +/- 5:5.

In preferred embodiments, the FePt particles are formed by contacting an iron salt, a platinum salt, and a reducing reagent. In certain embodiments, surfactant molecules and or other ligands are further added during particle synthesis to prevent agglomeration of the FePt particles formed. Suitable iron sources include, but are not limited to, iron salts such as Fe(II) acetylacetonate, Fe(III) acetylacetonate, Fe(II) chloride, Fe(III) chloride, Fe(II) acetate, Fe(II) bromide, Fe(III) bromide, Fe(II) fluoride, Fe(III) fluoride, Fe(II) iodide, and iron(II) sulphide. Suitable platinum sources include, but are not limited to, platinum salts such as Pt(II) acetylacetonate, Pt(II) acetate, Pt(II) chloride, Pt(II) bromide, Pt(II) iodide, and Pt(II) cyanide. In a preferred embodiment, the iron salt is Fe(III) acetylacetonate and the platinum salt is Pt(II) acetylacetonate. The relative amounts of iron salts and platinum salts may be selected based on the final desired Fe to Pt molar ratio composition of the FePt particle. Suitable reducing reagents include long chain diols such as, but not limited to, 1,2-hexadecanediol, 1,2-dodecanediol, and 1,2-octanediol. In a preferred embodiment, the reducing reagent is 1 ,2- hexadecanediol. Suitable surfactants may also be added and include, but are not limited to, oleic acid, oleylamine, hexanoic acid, dodecyl-benzene sodium sulfate, and sodium dodecylsulfonate. In a preferred embodiment, oleylamine and/or oleic acid are used as surfactants. The reaction to form the FePt particles may be performed at a suitable temperature in the range from about 100 °C to about 300 °C. The rate at which the reaction is heated, either to an intermediate temperature (if any), or to the temperature to which the reaction is ultimately heated, may affect the size of the particles. Typical heating rates may be between about 1 to about 20 °C/min. The reaction is typically carried out in the presence of one or more solvents, such as an organic solvent (i.e., dioctyl ether or phenyl ether), under inert atmosphere, for any suitable amount of time which may be required to produce the final desired FePt particles of a given composition, size, and shape. The final FePt particles may be purified, as necessary, according to any suitable technique known in the art.

The FePt particles have an average size in the range from about 10 to about 500 nm, more preferably from about 100 to about 300 nm. In some embodiments, the FePt particles formed may be substantially mono-disperse, wherein the term "mono-disperse" means that the standard deviation of the particle diameter over the average particle diameter is less than about 10 percent. The FePt particles prepared may have shapes selected from spherical, spheroid, rod, oblate ellipsoid, or other shapes. In some embodiments the particle shape is selected to increase the probability of higher order particle stacking and/or increased particle packing.

The FePt particles formed according to any of the methods described above may optionally include other metals such as, but not limited to, silver, cobalt, and nickel to increase the magnetization properties and/or improve the physical properties of the particles. For example, metal salts such as Co(II) acetylacetonate, Ag(I) acetate, and Ni(II) acetylacetonate may be added to substitute at least some of the Fe and/or Pt metal salts used in the synthesis of the particles.

Alternatively, in some embodiments, the FePt particles may be formed by decomposition of iron pentacarbonyl (Fe(CO)s) and *in situ* reduction of Pt(II) acetylacetonate at a high temperature in the range of about 250° C to about 300° C, according to methods known in the art.

### Annealing with Silica Coating

To reduce or prevent disintegration during annealing, the Fe/Pt particles are coated with a silica shell. Coating magnetic particles with silica reduces the formation of aggregates; enhances stability, decreases undesirable alterations in magnetic properties; and reduces biodegradation when used *in vivo* (Santra, Langmuir, 17:2900-06 (2001)). Methods of coating magnetic particles with silica are known in the art, and typically include microemulsions prepared with a non-ionic surfactant and tetraethylorthosilicate (TOES), followed by annealing. After annealing, silica can be removed with hydrofluoric acid. An exemplary method is provided in the examples.

The crystalline structure is an important feature. The FePt particles are coated with silica (SiO₂) by base-catalyzed silica formation, or some other suitable method known in the art, in order to reduce or inhibit thermal aggregation and/or disintegration of the FePt particles prior to applying an annealing treatment to the particles at a high temperature These particles, referred to as FePt@SiO₂ particles, may be isolated and purified according to any suitable technique known in the art.

The FePt@SiO₂ particles can be annealed at a temperature in the range from about 400 to about 1000 °C, most typically from about 600 to about 750 °C. In a preferred embodiment, the FePt@SiO₂ particles are annealed at about 700 °C. The FePt@SiO₂ may be purged with a mixture of one or more gases. In some embodiments, the purging gas is a reducing gas mixture (i.e., a mixture of hydrogen gas and nitrogen gas) during the annealing treatment. The annealing treatment is applied for an amount of time sufficient to induce an interior crystalline phase in the particles, which is an Ll₀ phase. In the preferred embodiment, the Fe/Pt is stabilized prior to annealing by application of a colloidal coating of thermally resistant inorganic materials such as silica, alumina powder, ceramics, iron oxides, titanium oxides, urethanes and epoxies. These shells allow for the formation of a unified FePt core and L1₀ ordering within each particle and prevent the coalescence of the FePt cores of adjacent particles. In a preferred embodiment the annealing step at 700 °C is carried out for about 30 minutes to about one hour. The annealing step may be performed in air, under the flow of a purging gas, or under inert atmosphere. Following the annealing treatment, the coating layer can be removed, and the FePt particles isolated and purified.

The magnetic properties of the as-synthesized and annealed FePt particles may be characterized using a sensitive magnetometer, such as a superconducting quantum interference scaffold (SQUID) to determine the coercivity from the magnetization curve of the particles. Coercivity, as used herein, is a measure of the resistance of a magnetic to becoming demagnetized. In preferred embodiments, the annealed FePt particles have a coercivity in the range of about 5,000 to about 25,000 amperes per meter (A/m), or in the range of about 0.05 to about 2.5 Tesla. High coercivity in the annealed particles may be attributed to L1₀ ordering in FePt particle induced by the annealing treatment.

These FePt magnetic particles may be neutral or negatively or positively charged. The FePt magnetic particles can have a zeta potential in the range from about -60 mV to about + 60 mV. In a preferred embodiment, the annealed FePt particles are uncharged.

The annealed FePt magnetic particles can be dispersed in a polymer solution, suspension, or emulsion that is used to coat or impregnate a scaffold, or such that when the polymer polymerizes, the magnetic particles are immobilized by the polymeric matrix in or on the scaffold, such as a stent. Alternatively, the annealed FePt particles may be conjugated to a polymer, such as an alginate. In other embodiments, the annealed FePt particles can be encapsulated non-covalently in a polymeric particle. In some embodiments, the polymeric particle is a particle having a diameter in the rage of about 100 to about 500 nm, most preferably about 300 to about 500 nm. In other embodiments, the polymeric particle is a particle having a diameter in the rage of about 500 nm to about 10 microns.

### Magnetizing the Particles

Typically, the particles are placed in a magnetic field to magnetize or re-magnetize them. The magnetic field can be that of a permanent magnet or an electromagnet. The strength and length of magnetism exhibited by the particles can be tuned by the strength of external magnet and duration used to magnetize is the particles. The magnetic field can be applied when the particles are *ex vivo, in vivo,* or a combination thereof. Preferably, the particles are of a suitable magnetic field/strength and duration to achieve the desired application.

The paramagnetic particles have an iron/platinum core. Previous versions were not annealed (i.e., not heated to create the L1₀ crystalline phase needed to hold a magnetic moment). As such they were superparamagnetic and thus displayed no hysteresis in the magnetization curve (i.e., not ferromagnetic). Annealing the Fe:Pt is very important for introducing a crystal structure LI0 interior crystalline phase. Annealing takes place at temperatures over 600°C. In a preferred embodiment, the particles are annealed at 700°C for 30 min. This creates the magnetization. Particle disintegration is minimized by coating the FePT with Silica then heating to prevent particle disintegration. The Fe:Pt molar ratio for creation of the crystal phase is important and an average compositional molar ratio of Fe to Pt is in the range 40:60 +/- 10: 10 mol%, and preferably +/- 5:5.

The magnetic force as a whole can be measured with a "Super Conducting Quantum Interference Scaffold", which is a sensitive magnetometer. The overall magnetic force is in the range from 0.1 to 2.0 Tesla.

Typically, the particles are placed in a magnetic field to magnetize or re-magnetize them. The magnetic field can be that of a permanent magnet or an electromagnet. In a particular embodiment, the particles are magnetized in a clinical scanner, for example a magnetic field generated by a Magnetic Resonance Imaging (MRI) scanner. The strength and length of magnetism exhibited by the particles can be tuned by the strength of external magnet and duration used to magnetize is the particles. The magnetic field can be applied when the particles are *in situ, ex vivo, in vivo,* or a combination thereof. It is preferred that the particles are applied to the device before magnetization.

Preferably, the particles are of a suitable magnetic field/strength and duration to achieve the desired application. For example, a magnetic field strength of between 0.1 and 5 T, or between 0.5 and 3 T could be used to magnetize the particles. The magnetic particle can be selected by the practitioner based on the desired properties including the strength and length of magnetism as discussed above and in more detail below. In certain therapeutic applications, it may be desirable for the particles to be ferromagnetic, i.e. maintain a magnetic field. For most *in vivo* applications, the magnetic field will be between 0.1 and 2.0 Tesla, more preferably between 0.05 and 0.3 Tesla. The particles preferably remain magnetic *in vivo* for at least between about 1 and 25, 1 and 50, 1 and 75, or between about 1 and 100 days, most preferably at least 60 days, each inclusive after removal from a magnetic field. For most *in vivo* applications, the magnetic field will be between 0.1 and 2.0 Tesla, more preferably between 0.05 and 0.3 Tesla. The FePt particles have magnetic retention of at least 60 days.

### B. Polymeric Scaffolds, Meshes and Bone Cements

### 1. Polymeric Meshes and Scaffolds

A polymeric matrix may be formed from non-biodegradable or biodegradable polymers; however, preferably, the polymeric matrix is biodegradable. The polymeric matrix can be selected to degrade over a time period ranging from one day to one year, more preferably from seven days to 26 weeks, more preferably from seven days to 20 weeks, most preferably from seven days to 16 weeks.

In general, synthetic polymers are preferred, although natural polymers may be used. Representative polymers include poly(lactic acid), poly(glycolic acid), poly(lactic acid-co-glycolic acids), polyhydroxyalkanoates such as poly3-hydroxybutyrate or poly4-hydroxybutyrate; polycaprolactones; poly(orthoesters); polyanhydrides; poly(phosphazenes); poly(lactide-co-caprolactones); poly(glycolide-co-caprolactones); polycarbonates such as tyrosine polycarbonates; polyamides (including synthetic and natural polyamides), polypeptides, and poly(amino acids); polyesteramides; other biocompatible polyesters; poly(dioxanones); poly(alkylene alkylates); hydrophilic polyethers; polyurethanes; polyetheresters; polyacetals; polycyanoacrylates; polysiloxanes; poly(oxyethylene)/poly(oxypropylene) copolymers; polyketals; polyphosphates; polyhydroxyvalerates; polyalkylene oxalates; polyalkylene succinates; poly(maleic acids), polyvinyl alcohols, polyvinylpyrrolidone; poly(alkylene oxides) such as polyethylene glycol (PEG); derivativized celluloses such as alkyl celluloses (*e.g*., methyl cellulose), hydroxyalkyl celluloses (*e.g*., hydroxypropyl cellulose), cellulose ethers, cellulose esters, nitrocelluloses, polymers of acrylic acid, methacrylic acid or copolymers or derivatives thereof including esters, poly(methyl methacrylate), poly(ethyl methacrylate), poly(butylmethacrylate), poly(isobutyl methacrylate), poly(hexylmethacrylate), poly(isodecyl methacrylate), poly(lauryl methacrylate), poly(phenyl methacrylate), poly(methyl acrylate), poly(isopropyl acrylate), poly(isobutyl acrylate), and poly(octadecyl acrylate) (jointly referred to herein as "polyacrylic acids"), as well as derivatives, copolymers, and blends thereof.

As used herein, "derivatives" include polymers having substitutions, additions of chemical groups and other modifications to the polymeric backbones described above routinely made by those skilled in the art. Natural polymers, including proteins such as albumin, collagen, gelatin, prolamines, such as zein, and polysaccharides such as alginate and pectin, may also be incorporated into the polymeric matrix. While a variety of polymers may be used to form the polymeric matrix, generally, the resulting polymeric matrix will be a hydrogel. In certain cases, when the polymeric matrix contains a natural polymer, the natural polymer is a biopolymer which degrades by hydrolysis, such as a polyhydroxyalkanoate.

Perhaps the most widely used are the aliphatic polyesters, specifically the hydrophobic poly(lactic acid) (PLA), more hydrophilic poly(glycolic acid) PGA and their copolymers, poly(lactide-co-glycolide) (PLGA). The degradation rate of these polymers, and often the corresponding drug release rate, can vary from days (PGA) to months (PLA) and is easily manipulated by varying the ratio of PLA to PGA. Second, the physiologic compatibility of PLGA and its homopolymers PGA and PLA have been established for safe use in humans; these materials have a history of over 30 years in various human clinical applications including drug delivery systems. PLGA particles can be formulated in a variety of ways that improve drug pharmacokinetics and biodistribution to target tissue by either passive or active targeting. The particles are designed to release molecules to be encapsulated or attached over a period of days to weeks. Factors that affect the duration of release include pH of the surrounding medium (higher rate of release at pH 5 and below due to acid catalyzed hydrolysis of PLGA) and polymer composition. Aliphatic polyesters differ in hydrophobicity and that in turn affects the degradation rate. Specifically the hydrophobic poly(lactic acid) (PLA), more hydrophilic poly (glycolic acid) PGA and their copolymers, poly(lactide-co-glycolide) (PLGA) have various release rates. The degradation rate of these polymers, and often the corresponding drug release rate, can vary from days (PGA) to months (PLA) and is easily manipulated by varying the ratio of PLA to PGA.

Examples of preferred natural polymers include proteins such as albumin, collagen, gelatin and prolamines, for example, zein, and polysaccharides such as alginate, cellulose derivatives and polyhydroxyalkanoates, for example, polyhydroxybutyrate. The *in vivo* stability of the particles can be adjusted during the production by using polymers such as poly(lactide-co-glycolide) copolymerized with polyethylene glycol (PEG). If PEG is exposed on the external surface, it may increase the time these materials circulate due to the hydrophilicity of PEG.

Examples of preferred non-biodegradable polymers include ethylene vinyl acetate, poly(meth)acrylic acid, polyamides, copolymers and mixtures thereof.

### 2. Hydrogel Polymeric Scaffolds

In some embodiments, the annealed FePt particles are conjugated to a hydrogel forming polymer. A hydrogel is a substance formed when an organic polymer (natural or synthetic) is cross-linked via covalent, ionic, or hydrogen bonds to create a three-dimensional open-lattice structure which entraps water molecules to form a gel. According to one embodiment, when the annealed FePt particles have a molar composition which is about 50% or greater in Fe content, the weight percentage of FePt magnetic particles in the hydrogel is in the range of about 1 - 50 wt%. In yet another embodiment, when the FePt particles have a molar composition which is about 50% or less in Fe content, the weight percentage of FePt magnetic particles in the hydrogel is chosen to be in the range of about 5 - 50 wt%.

### 3. Magnetic Hydrogel Depots and Grafts

Hydrogels are three-dimensional networks composed of hydrophilic polymers crosslinked either through covalent bonds or held together via physical intramolecular and intermolecular attractions. Hydrogels can absorb huge amounts of water or biological fluids, up to several thousand %, and swell readily without dissolving. The high hydrophilicity of hydrogels is particularly due to the presence of hydrophilic moieties such as carboxyl, amide, amino, and hydroxyl groups distributed along the backbone of polymeric chains. In the swollen state, hydrogels are soft and rubbery, resembling to a great extent the living tissues. In addition, many hydrogels, such as chitosan and alginate-based hydrogels show desirable biocompatibility. The appearance of hydrogels dates back more than fifty years, when poly(2-hydroxyethyl methacrylate)-based hydrogel was developed for contact lens applications. The uses of hydrogels have extended to cover a wide range of applications that include, but are not limited to, drug delivery, wound healing, ophthalmic materials and tissue engineering. Hydrogels usually reach their equilibrium swelling when a balance occurs between osmotic driving forces, which encourage the entrance of water or biological fluids into the hydrophilic hydrogel matrix, and the cohesive forces exerted by the polymer strands within the hydrogel. These cohesive forces resist the hydrogel expansion and the extent of these forces depends particularly on the hydrogel crosslinking density. In general, the more hydrophilic the polymer forming the hydrogel, the higher the total water amount absorbed by the hydrogel. Equally, the higher the crosslinking extent of a particular hydrogel, the lower the extent of the gel swelling.

Hydrogels can be classified into natural, synthetic and semi-synthetic according to their origin. Most of the synthetic hydrogels are synthesized by traditional polymerization of vinyl or vinyl-activated monomers. The equilibrium swelling values of these synthetic hydrogels vary widely according to the hydrophilicity of the monomers and the crosslinking density. A bi-functional monomer is usually added to carry out an *in situ* crosslinking reaction. Natural hydrogels are made of natural polymers including polynucleotides, polypeptides, and polysaccharides. These natural polymers are obtained from various natural origins. For instance, collagen is obtained from mammals whereas chitosan is obtained from shellfish exoskeletons.

Hydrogels can be either durable (such as most polyacrylate-based hydrogels) or biodegradable (such as polysaccharides-based hydrogels), depending on their stability characteristics in a physiological environment. The degradable polymers inside the hydrogel matrices undergo chain scission to form oligomers of low molecular weight. Then, the resulting oligomers are either eliminated by body or undergo further degradation. The effect of scaffold pore size on tissue regeneration, and optimum pore sizes for different purposes have been reported in many recent studies. For instance, it has been demonstrated that the optimum pore size for neovascularization is 5mm, 5- 15mm for ingrowth of fibroblast, 20 mm for hepatocytes ingrowth, 20- 125mm for regeneration of adult mammalian skin, and 200-350 mm for osteoconduction. The mechanical characteristics of hydrogels as scaffolds for tissue engineering can have a significant effect on either attached or encapsulated cells. One of the main parameters that control the mechanical compliance of hydrogel scaffolds is the crosslinking density, which can also be used to affect cells encapsulated within hydrogel networks. For instance, it has been reported that changes in the crosslinking density of PEG-based hydrogel caused changes in cell growth and morphology. Several hydrogels have been developed from natural polymers for tissue engineering applications. These natural polymers include for instance, polynucleotides, polypeptides, and different polysaccharides. They are obtained from a variety of natural origins; for example chitosan is obtained from shellfish exoskeletons whereas, collagen is obtained from mammals. Collagen hydrogel fibers are one of the most popular natural polymer-based hydrogel scaffolds in tissue engineering applications. These collagen hydrogel fibers are formed particularly through self-aggregation and crosslinking (through pyridinium crosslinks) of collagen molecules in a hydrated environment. In general, hydrogels based on polymers from natural origins such as collagen are advantageous in tissue engineering applications due to their intrinsic characteristics of biological recognition, including presentation of receptor-binding ligands and the susceptibility to cell-triggered proteolytic remodeling and degradation. However, the use of natural component-based hydrogels has shown some drawbacks, which involve the complexities associated with purification, immunogenicity and pathogen transmission. Synthetic polymer-based hydrogels were developed for different tissue engineering applications. Greater control over material characteristics and tissue responses are achievable when using hydrogels based on synthetic analogs. Hydrogel scaffolds based on self-assembled peptides (SAPs) are one of the main classes in tissue engineering applications. SAPs are polypeptides that undergo self-assembly under specific conditions, typically a hydrophilic environment, to form fibers or other types of nanostructures. These peptide-based amphiphilic molecules undergo self-assembly into a fibrous crosslinked hydrogel scaffold (arranged in ribbon-like parallel arrays). A variety of amphiphilic SAPs-based hydrogels have been used in various tissue engineering applications.

Emulsification is the most commonly used technique for fabricating hydrogel nano- and particles. Emulsification involves agitation of a multiphase mixture to generate small aqueous droplets of hydrogel precursors within a hydrophobic medium (such as oil or organic solvent). The droplets size can be controlled by the viscosity of the hydrogel precursor, the extent of mechanical agitation and through using of surfactants that can control the surface tension between the two phases as well as preventing aggregation of the resulting hydrogel particles. The hydrogel precursor droplets can be crosslinked using different crosslinking mechanisms to produce spherical nano- or microgels. Emulsification can be utilized to develop gel particles from a wide range of natural and synthetic polymers such as chitosan, polylactic acid, polylactic-co-glycolic acid, collagen, agarose and alginate. Cell-laden gel particles can be fabricated through the addition of cells to the aqueous phase containing the hydrogel precursor.

Lyophilization (freeze-drying) depends on the rapid cooling of a sample to produce thermodynamic instability within it, leading to a kind of phase separation. This is followed by sublimation of the solvent, leaving behind voids and pores. Representative materials include collagen-chitosan hydrogel scaffolds and agarose hydrogel scaffolds .

Solvent casting-leaching can be considered as the simplest technique for developing porous scaffolds with almost uniform pore size. The procedure includes the casting of an organic polymer solution containing a crosslinker and salt particulates, followed by solvent evaporation and dissolution of the entrapped salt particulates in water. Gas foaming-leaching involves using an effervescent salt as a gas foaming agent to develop the porous structure of the scaffolds. The scaffolds resulting from this approach showed a macro-porous open cellular structure with uniform pore sizes in the range of 100 to 200 mm. Chitosan, collagen, laminin, gelatin, matrigel, sodium alginate, and hyaluronic acid (hyaluronan) are the most commonly used natural polymers in developing hydrogels for cardiac tissue engineering applications. These natural polymers have structures very similar to the molecules in biological organisms, thus reducing the possibility of immune response when implanted in vivo. Synthetic polymers used for developing hydrogel matrices for cardiac tissue engineering include polylactide (PLA), polylactide-co-glycolic acid copolymer (PLGA), poly(ethylene glycol) (PEG), polycaprolactone (PCL), polyurethane (PU), and polyacrylamide (PAAm). The use of synthetic polymers is advantageous over natural polymers due to ease of tailoring their physicochemical characteristics, such as water affinity, modulus, and degradation rate, to meet the requirements of cardiac muscle tissue engineering.

The polymeric matrix may contain one or more crosslinkable polymers. Examples of suitable photo-polymerizable groups include vinyl groups, acrylate groups, methacrylate groups, and acrylamide groups. Photo-polymerizable groups, when present, may be incorporated within the backbone of the crosslinkable polymers, within one or more of the sidechains of the crosslinkable polymers, at one or more of the ends of the crosslinkable polymers, or combinations thereof.

According to one embodiment, the organic polymer which forms the hydrogel possesses one or more reactive functional groups along the polymer backbone which serve as conjugation sites for covalent coupling to reactive functional groups present on FePt particles. Exemplary reactive functional groups include, but are not limited to, carboxylic acid and activated derivatives thereof, amino, maleimide, thiol, sulfonic acid and derivatives thereof, carbonate and derivatives thereof, carbamate and derivatives thereof, hydroxyl, aldehyde, ketone, hydrazine , isocyanate, isothiocyanate, phosphoric acid and derivatives, phosphonic acid and derivatives, haloacetyl, alkyl halides, vinyl sulfone, vinyl ketone, epoxide, oxirane, and aziridine. The covalent functionalization of the hydrogel polymer reactive functional groups with annealed FePt particles containing reactive functional groups can be carried out through any of a number of chemistries available in the art to provide a degradable or non-degradable covalent bond between the hydrogel and the annealed FePt particle. In one example, the hydrogel may be formed of an alginate which contains pendant carboxylic acid groups along the backbone and FePt particles containing one or more pendant amino groups and are reacted via a standard amine-coupling reaction with, for example, N-hydroxysuccinimide (NHS) and a carbodiimide, (e.g., EDC or DCC). In a preferred embodiment, a hydrogel forming polymer is first covalently functionalized with annealed FePt particles and subsequently crosslinked to form the hydrogel.

The matrix can be made of polymers such as alginate, produced through traditional ionic gelation techniques. The polymers are first dissolved in an aqueous solution, mixed with barium sulfate or some bioactive agent, and then extruded through a microdroplet forming scaffold, which in some instances employs a flow of nitrogen gas to break off the droplet. A slowly stirred (approximately 100-170 RPM) ionic hardening bath is positioned below the extruding scaffold to catch the forming microdroplets. The particles are left to incubate in the bath for twenty to thirty minutes in order to allow sufficient time for gelation to occur. Particle size is controlled by using various size extruders or varying either the nitrogen gas or polymer solution flow rates. Chitosan particles can be prepared by dissolving the polymer in acidic solution and crosslinking it with tripolyphosphate. Carboxymethyl cellulose (CMC) particles can be prepared by dissolving the polymer in acid solution and precipitating the particle with lead ions. In the case of negatively charged polymers (e.g., alginate, CMC), positively charged ligands (e.g., polylysine, polyethyleneimine) of different molecular weights can be ionically attached.

### C. Therapeutic, Prophylactic and Diagnostic Agents

The surface of the particle can be modified through the creation of a few atomic layers of organic (polymer) or inorganic (metal or oxide) surfaces, and is then suitable for further functionalization with therapeutic, prophylactic and/or diagnostic agents. These may be small molecule active agents or biomacromolecules, such as proteins, polypeptides, or nucleic acids. Suitable small molecule active agents include organic and organometallic compounds. The small molecule active agents can be a hydrophilic, hydrophobic, or amphiphilic compound. It may also be advantageous to incorporate onto or into the particle, a contrast agent, radiopaque markers, fluorescent dye, or other additives to allow the particles to be imaged *in vivo* for tracking, positioning, and other purposes.

In some embodiments, the magnetic particles are dispersed in a polymer solution, suspension, or emulsion that is used to coat or impregnate a scaffold, or such that when the polymer polymerizes, the magnetic particles are immobilized by the polymeric matrix into or onto the scaffold. Therapeutic, prophylactic and diagnostic agents can be added to the solution, suspension, or emulsion so that the active agent is also incorporated in the polymeric coating.

Therapeutic agents, include, but not limited to, antiplatelet agents, anticoagulant agents, anti-inflammatory agents antimicrobial agents, antimetabolic agents, additional anti-neointima agents, additional antiproliferative agents, immunomodulators, antiproliferative agents, agents that affect migration and extracellular matrix production, agents that affect platelet deposition or formation of thrombis, and agents that promote vascular healing and re-endothelialization, such as those and others described in Tanguay et al. Cardiology Clinics, 12:699-713 (1994), J. E. Sousa, et al., Circulation, 107 (2003) 2274 (Part I), 2283 (Part II), Salu, et al., Acta Cardiol, 59 (2004) 51.

Examples of antithrombin agents include, but are not limited to, Heparin (including low molecular heparin), R-Hirudin, Hirulog, Argatroban, Efegatran, Tick anticoagulant peptide, and Ppack.

Examples of antiproliferative agents include, but are not limited to, Paclitaxel (Taxol), QP-2 Vincristin, Methotrexat, Angiopeptin, Mitomycin, BCP 678, Antisense c-myc, ABT 578, Actinomycin-D, RestenASE, 1 - Chlor-deoxyadenosin, PCNA Ribozym, and Celecoxib.

Examples of anti-restenosis agents include, but are not limited to, immunomodulators such as Sirolimus (Rapamycin), Tacrolimus, Biorest, Mizoribin, Cyclosporin, Interferon-γ 1b, Leflunomid, Tranilast, Corticosteroide, Mycophenolic acid and Biphosphonate.

Examples of anti-migratory agents and extracellular matrix modulators include, but are not limited to Halofuginone, Propyl-hydroxylase-Inhibitors, C-Proteinase-Inhibitors, MMP-Inhibitors, Batimastat, Probucol.

Examples of wound healing agents and endothelialization promoters include vascular epithelial growth factor ("VEGF"), 17β-Estradiol, Tkase-Inhibitors, BCP 671, Statins, nitric oxide ("NO")-Donors, and endothelial progenitor cell ("EPC")-antibodies.

Contrast agents such as radiopaque markers, or other additives to allow the stent to be imaged *in vivo* for tracking, positioning, and other purposes can also be incorporated in the stent. Such additives could be added to the absorbable composition used to make the stent or stent coating, or absorbed into, melted onto, or sprayed onto the surface of part or all of the stent. Preferred additives for this purpose include silver, iodine and iodine labeled compounds, barium sulfate, gadolinium oxide, bismuth derivatives, zirconium dioxide, cadmium, tungsten, gold tantalum, bismuth, platinum, iridium, and rhodium. These additives may be, but are not limited to, micro- or nano-sized particles or particles. The particles can be the same or different from the magnetized particles discussed above. Radio-opacity may be determined by fluoroscopy or by x-ray analysis. Imaging and contrast enhancing modifications, such as conjugation of iodine to the particle, are discussed in more detail below.

### II. Methods of Coating and Impregnating Scaffolds

The magnetic particles can be coated onto or impregnated into the scaffold using any suitable means. Common scaffold-coating methods include, for example, ion beam deposition, chemical vapor deposition, plasma vacuum technology, atomization, dipping, ultrasound, inkjet printing, gas phase deposition, electro-spinning, and electro-spraying. In particularly preferred embodiments, a polymer/particle solution, suspension, or emulsion applied to the scaffold by a spray-based method such as electro-spray, or electro-nanospray.

The thickness of the polymeric coat also depends on the intended use. Preferably the coat is between about 1µm and 1,000µm inclusive, or between about 5µm and 500µm inclusive, or between about 10µm and 100µm inclusive. In a particular embodiment, the coat thickness is about 10 µm, 25 µm, 50 µm, or 75 µm. Magnesium stent coatings are preferably 40 to 60 microns in thickness.

Preferably, the particles make up between about 1% and 30% by weight, magnetic particles to polymer for greater than 50% Fe Fe/Pt particles and between 5 and 30% by weight magnetic particles to polymer for less than 50% Fe Fe/Pt particles.

Most typically, the scaffold will have an effective magnetic field and remain magnetized for a sufficient amount of time to attract an effective amount of magnetized cells to the target site to enhance a function of the tissue and/or treat a tissue injury at the target site.

As discussed in more detail, the materials and methods disclosed herein are particularly suitable to enhancing repair of injury to cardiac and vascular tissue and other tissues exposed to forces and stresses caused by biologic fluid flow. Therefore, in preferred embodiments, the scaffold will have an effective magnetic field and remain magnetized for a sufficient amount of time to attract, capture, and/or an retain an effective amount of magnetized cells to the target site to enhance repair of tissue injury. Preferably, the scaffold will have an effective magnetic field and remain magnetized for a sufficient amount of time to attract, capture, and/or retain an effective amount of magnetized cells to the target site for 1, 2, 3, 4, 5, 6, 7, or more days, weeks, or months, most preferably 60 days for cardiovascular applications.

In some embodiments, the magnetic particles are conjugated to a polymer or gelling agent, or otherwise incorporated into or dispersed within a hydrogel prior to crosslinking, such that the crosslinking forms a hydrogel depot or graft containing the magnetic particles. In preferred embodiments, this is accomplished by linking, for example, covalently linking, magnetic particles to the polymer or gelling agent of the hydrogel. The magnetic particles can be functionalized with reactive groups that can link to the polymer or gelling agent using conventional methods.

In a preferred embodiment, the linking of the magnetic particle to the polymer or gelling agent is by carboxyl-to-amine conjugation. For example, the magnetic particles can be functionalized with diethylene triamine pentaacetic acid (DTPA) to add primary amines that can be coupled to carboxyl groups on the gelling agent. In a particular embodiment, the carboxyl-to-amine conjugation is carried out using a water-soluble carbodiimide crosslinker such as 1-Ethyl-3-[3-dimethylaminopropyl]carbodiimide hydrochloride (EDC or EDAC) is used alone or in combination with N-hydroxysulfosuccinimide (Sulfo-NHS) or its uncharged analog N-hydroxysuccinimide (NHS) which can increase reaction efficiency and/or stabilize active intermediates for later reaction to amines. In some embodiments, the particles or hydrogels are also functionalized with a label, for example iodine as discussed below, to yield hydrogels that can be monitored *in vivo.*

In a particular embodiment exemplified below, magnetic Fe/PT particles are functionalized with DTPA and cross-linked to polyalginate with EDC prior to gelation.

### A. Gelling Agents

Although exemplified with alginate, other gelling agents are also contemplated. Some of the common ones are acacia, alginic acid, bentonite, Carbopols^{®} (now known as carbomers), carboxymethylcellulose. ethylcellulose, gelatin, hydroxyethylcellulose, hydroxypropyl cellulose, magnesium aluminum silicate (Veegum^{®}), methylcellulose, poloxamers (PLURONICs^{®}), polyvinyl alcohol, sodium alginate, tragacanth, and xanthan gum. Though each gelling agent has some unique properties, there are some generalizations that can be made.

Some gelling agents are more soluble in cold water than in hot water. Methylcellulose and poloxamers have better solubility in cold water while bentonite, gelatin, and sodium carboxymethylcellulose are more soluble in hot water. Carbomers, tragacanth, and alginic acid gels are made with tepid water.

Some gelling agents (carbomers) require a "neutralizer" or a pH adjusting chemical to create the gel after the gelling agent has been wetted in the dispersing medium.

Most gelling agents require 24-48 hours to completely hydrate and reach maximum viscosity and clarity. Gelling agents are used in concentrations of 0.5% up to 10% depending on the agent.

### 1. Carbomer

Carbomer is a generic name for a family of polymers known as Carbopol^{®}. Carbopols^{®} were first used in the mid 1950s. As a group, they are dry powders with high bulk densities, and form acidic aqueous solutions (pH around 3.0). They thicken at higher pHs (around 5 or 6). They will also swell in aqueous solution of that pH as much as 1000 times their original volume. Their solutions range in viscosity from 0 to 80,000 centipoise (cps, 1cps corresponds to 1 mPa·s). Some examples of this group of gelling agents are:
Carbopol^{®} 910 has viscosity of 3,000 - 7,000 cps and is effective in low concentrations and provides a low viscosity formulation;
Carbopol^{®} 934 has a viscosity of 30,500 - 39,400 cps and is effective in thick formulations such as emulsions, suspensions, sustained-release formulations, transdermals, and topicals;
Carbopol^{®} 934P has a viscosity of 29,400 - 39,400 cps with the same properties as 934, but is intended for pharmaceutical formulations;
Carbopol^{®} 940 has a viscosity of 40,000 - 60,000 cps and is effective in thick formulations, has very good clarity in water or hydroalcoholic topical gels; and Carbopol^{®} 941 has a viscosity of 4,000 - 11,000 cps and produces low viscosity gels with very good clarity.

Carbomer polymers are best introduced into water by slowly sprinkling a sieved powder into the vortex created by rapid stirring. This should prevent clumping. Once all of the powder has been added, the stirring speed should be reduced to decrease the possibility of entrapping air bubbles in the formulation.

As mentioned, when the carbomer is dispersed, the solution will have a low pH. A "neutralizer" is added to increase the pH and cause the dispersion to thicken and gel. Some neutralizing agents are sodium hydroxide, potassium hydroxide, and triethanolamine. If the inorganic bases are used to neutralize the solution, a stable water soluble gel is formed. If triethanolamine is used, the gel can tolerate high alcohol concentrations. The viscosity of the gel can be further manipulated by propylene glycol and glycerin (to increase viscosity) or by adding electrolytes (to decrease viscosity).

### 2. Cellulose Derivatives

The cellulose derivatives (methylcellulose, hydroxyethylcellulose, hydroxypropylcellulose, hydroxypropylmethylcellulose, and carboxymethylcellulose) are commonly used. There are some commonalties in these compounds, and each one has their unique properties.

Methylcellulose has a viscosity of 1500 cps and makes thinner gels with high tolerance for added drugs and salts. It is compatible with water, alcohol (70%), and propylene glycol (50%) and hydrates and swells in hot water. The powder is dispersed with high shear in about 1/3 of the required amount of water at 80°C to 90°C. Once it is dispersed, the rest of the water (as cold water or ice water) is added with moderate stirring. Maximum clarity, hydration, and viscosity will be obtained if the gel is cooled to 0-10°C for about an hour.

Hydroxyethylcellulose makes thinner gels that are compatible with water and alcohol (30%). It hydrates and swells in cool water (about 8-12 hours). It forms an occlusive dressing when lightly applied to the skin and allowed to dry

Hydroxypropylcellulose makes thinner gels with high tolerance for added drugs and salts and is compatible with alcohols and glycols. It hydrates and swells in water or hydroalcoholic solution. The powder is sprinkled in portions into water or hydroalcoholic solution without stirring and allowed to thoroughly wet. After all of the powder is added and hydrated (about 8-12 hours), the formulation can be stirred or shaken. It is a good gelling agent if 15% or more of an organic solvent is needed to dissolve the active drug.

Hydroxypropylmethylcellulose makes thicker gels but has a lower tolerance for positively charged ions. It is compatible with water, alcohol (80%) and disperses in cool water. It is a good gelling agent for time released formulations.

Carboxymethylcellulose is generally used as the sodium salt. It makes thicker gels but has less tolerance than hydroxypropylmethylcellulose. It has a maximum stability at pH 7 - 9 and is compatible with water and alcohol. It disperses in cold water to hydrate and swells. It is then heated to about 60°C. Maximum gelling occurs in 1 - 2 hours.

Poloxamer (PLURONICs^{®}) are copolymers of polyoxyethylene and polyoxypropylene. They will form thermoreversible gels in concentration ranging from 15% to 50%. This means they are liquids at cool (refrigerator) temperature, but are gels at room or body temperature. Poloxamer copolymers are white, waxy granules that form clear liquids when dispersed in cold water or cooled to 0-10°C overnight.

PLURONIC^{®} F-127 is often combined with a lecithin and isopropyl palmitate solution to make what is called a "PLO gel." This is a slight misnomer, since the final product is actually an emulsion. The confusion comes from using a gel as one of the ingredients for the emulsion. A syringe adaptor "PLO gel" is made by combining a PLURONIC^{®} F-127 gel and a lecithin/isopropyl palmitate syrup. The two components are made and stored separately. When it is time to compound a formulation, water soluble drugs are dissolved in the PLURONIC^{®} gel or oil soluble drugs are dissolved in the lecithin syrup. If a small quantity of formulation is to be made, each of the components can be put into a syringe and the two syringes are connected by a adapter. The mixture is forced between the two syringes and the shear caused by the passing the mixture through the adapter will create the "PLO gel."

### 3. Ionic Hydrogels

Ionic polysaccharides, such as alginates or chitosan, can be used. In one embodiment, the hydrogel is produced by cross-linking the anionic salt of alginic acid, a carbohydrate polymer isolated from seaweed, with ions, such as calcium cations. The strength of the hydrogel increases with either increasing concentrations of calcium ions or alginate. For example, U.S. Pat. No. 4,352,883 describes the ionic cross-linking of alginate with divalent cations, in water, at room temperature, to form a hydrogel matrix.

Magnetic particles are reacted with polyalginate solution for form magnetic polyaginate. The solution is delivered to a desired site in a subject and then solidifies in a short time due to the presence *in vivo* of physiological concentrations of calcium ions. Alternatively, the solution is delivered to the support structure prior to implantation and solidified in an external solution containing calcium ions. In addition, the support structure itself can be coated with or contain the appropriate ions, for example, calcium cations, to cause an ionic hydrogel to solidify once introduced into the support structure.

In general, these polymers are at least partially soluble in aqueous solutions, e.g., water, or aqueous alcohol solutions that have charged side groups, or a monovalent ionic salt thereof. There are many examples of polymers with acidic side groups that can be reacted with cations, e.g., poly(phosphazenes), poly(acrylic acids), and poly(methacrylic acids). Examples of acidic groups include carboxylic acid groups, sulfonic acid groups, and halogenated (preferably fluorinated) alcohol groups. Examples of polymers with basic side groups that can react with anions are poly(vinyl amines), poly(vinyl pyridine), and poly(vinyl imidazole).

Polyphosphazenes are polymers with backbones consisting of nitrogen and phosphorus atoms separated by alternating single and double bonds. Each phosphorus atom is covalently bonded to two side chains. Polyphosphazenes that can be used have a majority of side chains that are acidic and capable of forming salt bridges with di- or trivalent cations. Examples of acidic side chains are carboxylic acid groups and sulfonic acid groups.

Polyphosphazenes that erode *in vivo* have at least two different types of side chains: acidic side groups capable of forming salt bridges with multivalent cations, and side groups that hydrolyze under in vivo conditions, e.g., imidazole groups, amino acid esters, glycerol, and glucosyl. Degradable polymers, i.e., polymers that dissolve or degrade within a period that is acceptable in the desired application (usually *in vivo* therapy), will degrade in less than about five years and most preferably in less than about one year, once exposed to a physiological solution of pH 6-8 having a temperature of between about 25 °C and 38 °C. Hydrolysis of the side chain results in erosion of the polymer. Examples of hydrolyzing side chains are unsubstituted and substituted imidizoles and amino acid esters in which the side chain is bonded to the phosphorous atom through an amino linkage.

Methods for synthesis and the analysis of various types of polyphosphazenes are described in U.S. Pat. Nos. 4,440,921, 4,495,174, and 4,880,622. Methods for the synthesis of the other polymers described herein are known to those of ordinary skill in the art. See, for example Concise Encyclopedia of Polymer Science and Engineering, J. I. Kroschwitz, editor (John Wiley and Sons, New York, N.Y., 1990). Many polymers, such as poly(acrylic acid), alginates, and PLURONICS^{™}, are commercially available. Water soluble polymers with charged side groups are cross-linked by reacting the polymer with an aqueous solution containing multivalent ions of the opposite charge, either multivalent cations if the polymer has acidic side groups, or multivalent anions if the polymer has basic side groups. Cations for cross-linking the polymers with acidic side groups to form a hydrogel include divalent and trivalent cations such as copper, calcium, aluminum, magnesium, and strontium. Aqueous solutions of the salts of these cations are added to the polymers to form soft, highly swollen hydrogels.

Anions for cross-linking the polymers to form a hydrogel include divalent and trivalent anions such as low molecular weight dicarboxylate ions, terepthalate ions, sulfate ions, and carbonate ions. Aqueous solutions of the salts of these anions are added to the polymers to form soft, highly swollen hydrogels, as described with respect to cations.

### 4. Temperature-Dependent Hydrogels

Temperature-dependent, or thermosensitive, hydrogels can be used. These hydrogels have so-called "reverse gelation" properties, i.e., they are liquids at or below room temperature, and gel when warmed to higher temperatures, e.g., body temperature. Thus, these hydrogels can be easily applied at or below room temperature as a liquid and automatically form a semi-solid gel when warmed to body temperature. Examples of such temperature-dependent hydrogels are PLURONICS^{™} (BASF-Wyandotte), such as polyoxyethylene-polyoxypropylene F-108, F-68, and F-127, poly (N-isopropylacrylamide), and N-isopropylacrylamide copolymers.

These copolymers can be manipulated by standard techniques to alter physical properties such as their porosity, rate of degradation, transition temperature, and degree of rigidity. For example, the addition of low molecular weight saccharides in the presence and absence of salts affects the lower critical solution temperature (LCST) of typical thermosensitive polymers. In addition, when these gels are prepared at concentrations ranging between 5 and 25% (W/V) by dispersion at 4 °C, the viscosity and the gel-sol transition temperature are affected, the gel-sol transition temperature being inversely related to the concentration. These gels have diffusion characteristics capable of allowing cells to survive and be nourished.

U.S. Pat. No. 4,188,373 describes the use of PLURONIC^{™} polyols in aqueous compositions to provide thermal gelling aqueous systems. U.S. Pat. Nos. 4,474,751, '752, '753, and 4,478,822 describe drug delivery systems that utilize thermosetting polyoxyalkylene gels. With these systems, both the gel transition temperature and/or the rigidity of the gel can be modified by adjusting the pH and/or the ionic strength, as well as by the concentration of the polymer.

### 5. pH-Dependent Hydrogels

Other suitable hydrogels are pH-dependent. These hydrogels are liquids at, below, or above specific pH values, and gel when exposed to specific pH values, e.g., 7.35 to 7.45, which is the normal pH range of extracellular fluids within the human body. Thus, these hydrogels can be easily administered as a liquid and automatically form a semisolid gel when exposed to body pH. Examples of such pH-dependent hydrogels are TETRONICS^{™} (BASF-Wyandotte) polyoxyethylene-polyoxypropylene polymers of ethylene diamine, poly(diethyl aminoethyl methacrylate-g-ethylene glycol), and poly(2-hydroxymethyl methacrylate). These copolymers can be manipulated by standard techniques to affect their physical properties.

### 6. Light Solidified Hydrogels

Other hydrogels that can be used are solidified by either visible or ultraviolet light. These hydrogels are made of macromers including a water soluble region, a biodegradable region, and at least two polymerizable regions as described in U.S. Pat. No. 5,410,016. For example, the hydrogel can begin with a biodegradable, polymerizable macromer including a core, an extension on each end of the core, and an end cap on each extension. The core is a hydrophilic polymer, the extensions are biodegradable polymers, and the end caps are oligomers capable of cross-linking the macromers upon exposure to visible or ultraviolet light, e.g., long wavelength ultraviolet light.

Examples of such light solidified hydrogels include polyethylene oxide block copolymers, polyethylene glycol polylactic acid copolymers with acrylate end groups, and 10K polyethylene glycol-glycolide copolymer capped by an acrylate at both ends. As with the PLURONIC^{™} hydrogels, the copolymers comprising these hydrogels can be manipulated by standard techniques to modify their physical properties such as rate of degradation, differences in crystallinity, and degree of rigidity. Light solidified hydrogels are useful, for example, for direct painting of the hydrogel-cell mixture onto damaged tissue.

Regardless of type, a hydrogel should be biocompatible, biodegradable, and, preferably, able to solidify rapidly *in vivo* (i.e., in about 5 minutes or so following delivery to the support structure).

### 7. Shape Deformable Hydrogels

As described by Thornton, et al. Transplantation 77(12): 1798-803 (2004), shape-defining scaffolds for minimally invasive tissue engineering can also be used. Minimally invasive surgical procedures are increasingly important in medicine, but biomaterials consistent with this delivery approach that allow one to control the structure of the material after implantation are lacking. Biomaterials with shape-memorizing properties could permit minimally invasive delivery of cell transplantation constructs and enable the formation of new tissues or structures in vivo in desired shapes and sizes.

Macroporous alginate hydrogel scaffolds were prepared in a number of predefined geometries, compressed into significantly smaller, different "temporary" forms, and introduced into a site in need thereof. Scaffolds are rehydrated *in situ* with a suspension of cells or cell-free medium and delivered through the same catheter. Scaffolds recovered their original shape and size within 1 hr. The rapid recovery of scaffold properties facilitates efficient cell seeding in vivo and permits neotissue formation in desired geometries.

### IV. Magnetically Attractable Cells and Other Bioactive Agents

As discussed in more detail below, the disclosed magnetic materials are used to attract, capture, and/or retain target cells at a target site in need of cell therapy, *in vivo* in subject in need thereof. Typically, the cells are tagged or labeled with a magnetic or magnetically attractable material whereby they are attracted to the magnetic field exhibited by the magnetic materials. Additionally, or alternatively, the disclosed magnetic materials can be used to attract, capture, and/or retain magnetic particles functionalized with one or more bioactive agents.

### A. Cells

In the most preferred embodiments, the scaffolds are used in combination with magnetically attractable cells. Suitable cells include, but are not limited to, primary cells and established cell lines, immune cells, stem cells, and differentiated cells including, but not limited to, cells derived from ectoderm, endoderm, and mesoderm, including fibroblasts, parenchymal cells, hematopoietic cells, epithelial cells, mesenchymal cells, neural cells, endothelial cells, myoblasts, chondrocytes, osteoblasts, osteoclasts, bone marrow cells, stem cells, umbilical cord blood cells, or a combination thereof. As used herein, stem cells include unipotent cells, multipotent cells, and pluripotent cells; embryonic stem cells, and adult stem cells such as hematopoietic stem cells, mesenchymal stem cells, epithelial stem cells, and muscle satellite cells. The cells can be induced pluripotent stem cells (iPSCs).

The cells can be autologous or allogeneic cells. The autologous cells may be those naturally occurring in the donor or cells modified *ex vivo.* For example, in some embodiments, the cells have been recombinantly modified to contain one or more exogenous nucleic acids encoding desired protein products. In some embodiments, the cells are stem cells isolate from a donor and expanded and/or differentiated *ex vivo.*

A large body of evidence indicates that cells expressing the surface markers CD133 and CD34 constitute a phenotypically and functionally distinct population of circulating EPCs that may play a role in regenerative angiogenesis. CD34+ cells can be isolated from an available human source known to be enriched in progenitor cells (human umbilical cord blood), and differentiated into functional endothelial cells.

### Magnetic Labeling of Cells

Any suitable magnetic or magnetically attractable materials can be used to label the cells. Preferably the material is biocompatible and is not toxic to the cells or to the subject for which the therapy is intended. Magnetic and magnetically attractable cells and methods for preparing them are known in the art. See, for example, Nkansah, et al., Magn Reson Med., 65(6): 1776-1785 (2011), as well as references cited therein. Methods may include incubating the cells with the magnetic material under conditions suitable to be internalized by the cell. For example, in some embodiments, the magnetic material is internalized by endocytosis or pinocytosis. Antibodies tagged with fluorophores or magnetic beads, such as iron oxide, can be attached to target cells based on specific antibody/antigen recognition, which allows immunolabeling-based cell separation using flow cytometry or magnetic-activated cell sorting. In some cases, it is also possible to magnetically label cells without using particles.

One of the earliest works in magnetic capturing using bulk magnets uses a Magnetic Cell Sorter (MACS) from Miltenyi Biotec to separate cells labeled with magnetic particles from non-labeled cells. Three basic steps can be observed: the objects of interest are labeled with magnetic particles; the solution passes through the MACS Column, in which the labeled cells are captured by the magnets while the others are collected on the outlet of the column; the captured cells are removed from the action range of the magnetic field and collected. The same principle was used by Hoshino et al. to develop microfluidic systems in which bulk magnets with antiparallel magnetization are disposed side by side in order to create a higher field gradient. K. Hoshino, et al., Lab on Chip, 11, 3449-3457, 2011. This system is used to capture magnetically labeled cancer cells and to observe them inside the microfluidic channel.

Several companies such as Miltenyi Biotec, Dynal Biotech, Polysciences, Ademtech, or Chemicell have developed superparamagnetic particles of controlled size, coated with specific antibodies and dedicated to biomagnetic separation. Some of these particles are even composed of biodegradable materials, lowering their impact on cells.

Immunomagnetic enrichment of cells can be performed using different commercial equipment, such as the MACS^{®} system (Miltenyi Biotec), CELLSEARCH^{®} system (formerly Veridex, Warren, NJ, available through Janssen Diagnostics, LLC), and MPC separator series (Dynal AS). Recently reported approaches based on the combination of magnetism and microfluidics have also emerged as viable high throughput and low cost alternatives to powerful but bulky and expensive separation equipment such as the FACS (Fluorescence Activated Cell Sorter) or CELLSEARCH^{®} systems. A commonly used strategy consists in placing a bulk permanent magnet in the vicinity of a microfluidic channel to deflect magnetically labeled targets out of the main stream. The CELLSEARCH^{®} system, is the preferred system.

Suitable materials for labeling cells include magnetic particles such as the magnetic particles discussed in detail above. Particular embodiments, include iron oxide-based cell labeling, for example, ferumoxides or dextran-coated small particles of iron oxide (SPIOs), which have been used clinically to help identify tumors in the liver (Nkansah, et al., Magn Reson Med., 65(6): 1776-1785 (2011)). A clinically approved ferumoxide formulation is FERIDEX^{®}. Commercially available micron-sized iron oxide particles (MPIOs, Bangs Labs) have also been used for magnetic cell labeling Shapiro, et al., Magnetic Resonance in Medicine, 53(2):329-338 (2005). Biodegradable, polymer encapsulated magnetic particles, using polymers such as poly(lactide-co-glycolide) (PLGA) and poly(lactic acid) (PLA), have been prepared most typically for targeted delivery of encapsulated drug payloads and imaging confirmation (Nkansah, et al., Magn Reson Med., 65(6): 1776-1785 (2011)). Microgel iron oxide particles with a wide range of hydrodynamic diameters (86-766 nm) and substantial magnetite content (up to 82 wt%) for labeling endothelial progenitor cells are discussed in (Lee, et al., Biomaterials, 31(12):3296-3306 (2010)). 100 nm biodegradable poly(DL-lactic acid-co-α,β-malic acid/magnetite particles for magnetic cell labeling are discussed in Wang, et al., Biomaterials, 31(13):3502-3511 (2010) and magnetite cores encapsulated within PLGA at ~ 150 nm total diameter are discussed in Lim, et al., Small, 4(10):1640-1645 (2008). Cells may be labeled with magnetic and fluorescent or x-ray imageable, biodegradable micro- and particles, composed either of PLGA or cellulose. Methods of preparing such labeled cells are described in Nkansah, et al., Magn Reson Med., 65(6): 1776-1785 (2011). Other suitable compositions and methods are described in Arbab, NMR in Biomedicine, 18(6):383-389 (2005), Arbab, et al., Molecular Imaging, 3(1):24-32 (2004), and Hsiao, Magnetic Resonance in Medicine, 58(4):717-724 (2007).

### Pharmaceutical Compositions for Cells

The cells can be administered to the subject in a pharmaceutical composition. In general, pharmaceutical compositions include effective amounts of cells and optionally include pharmaceutically acceptable diluents, typically Dulbecco's phosphate buffered saline (PBS), Ringer's solution, 5% dextrose in water (D5W), and normal/physiologic saline (0.9% NaCl). Electrolytes such as, but not limited to, sodium chloride and potassium chloride may also be included in the therapeutic composition. Preferably, the pharmaceutical composition has a pH in a range from about 6.8 to about 7.4. In still another embodiment, the pharmaceutical composition has a pH of about 7.4. A wide variety of suitable formulations of pharmaceutical composition are known (see, e.g., Remington's Pharmaceutical Sciences, 22nd ed. 2012)).

### V. Methods of Administration (not claimed)

The magnetic scaffolds can be implanted into or otherwise administered to a subject to attract magnetic or magnetically attractable cells, magnetically attractable particles functionalized with active agents, and combinations thereof. Most typically, the scaffold is implanted at a site in need of cell therapy such that magnetic or magnetically attractable cells separately administered to the subject are magnetically attracted to the site *in vivo.* Additionally, or alternatively, magnetic or magnetically attractable cells can be seeded on or in the scaffold with cell *ex vivo* and the magnetic field produced by the scaffold retains the cells on or near the scaffold after it is implanted in a subject *in vivo.* In some *in vivo* approaches, the cells are administered to a subject in a therapeutically effective amount. As used herein the term "effective amount" or "therapeutically effective amount" means a dosage sufficient to treat, inhibit, or alleviate one or more symptoms of the disorder being treated or to otherwise provide a desired pharmacologic and/or physiologic effect. For example, the cells can be administered in an effective amount to enhance a tissue function. In preferred embodiments, cells are administered in an effective amount to enhance tissue repair from injury.

In some embodiments, the effect of the composition on a subject is compared to a control. For example, the effect of the composition on a particular symptom, pharmacologic, or physiologic indicator can be compared to an untreated subject, or the condition of the subject prior to treatment. In some embodiments, the symptom, pharmacologic, or physiologic indicator is measured in a subject prior to treatment, and again one or more times after treatment is initiated. In some embodiments, the control is a reference level, or average determined based on measuring the symptom, pharmacologic, or physiologic indicator in one or more subjects that do not have the disease or condition to be treated (e.g., healthy subjects). In some embodiments, the effect of the treatment is compared to a conventional treatment that is known the art, such as one of those discussed herein.

Cells are preferably administered by injection or catheter, parenterally or intravenously. In certain embodiments, the compositions are administered locally, for example by injection directly into or adjacent to a site to be treated. In some embodiments, the compositions are injected, topically applied, or otherwise administered directly into the vasculature or onto vascular tissue at or adjacent to a site of injury, surgery, or implantation. For example, the compositions are topically applied to vascular tissue that is exposed, during a surgical or implantation, or transplantation procedure. Typically, local administration causes an increased localized concentration of the compositions which is greater than that which can be achieved by systemic administration.

The disclosed materials and cells can be used in various combinations in a wide range of therapeutic applications based on the subject to be treated and the disease or disorder to be treated.

### VI. Treatments (not claimed)

The materials and methods are particularly useful for increasing, enhancing, or improving the tissue function, providing mechanical support and promoting tissue healing and repair processes. The materials and methods can also be effective to reduce, alleviate, or relieve, one or more symptoms of a disease or disorder associated with a damaged tissue. In addition, the term treatment includes prevention or postponement of development of diseases or disorders associated with a damaged tissue.

In particular embodiments, the tissue is a vascular tissue, a myocardial tissue, a muscle tissue, a kidney tissue, a cartilage tissue, a bone tissue, or a dermal tissue. The damaged tissue can be one which is functionally and/or structurally impaired, such as, but not limited to, an injured or restenoic endothelium, infarcted (post MI) myocardium, an ischemic myocardium, an ischemic muscle, an ischemic cartilage, an ischemic bone or an ischemic dermis. In other embodiments, the material is a bone cement, a tissue bulking agent, a prosthetic device such as a heart valve, breast or bladder reconstructive mesh, skull or facial reconstruction/plastic surgery device, dental implant, dental cement, drug delivery device, anti-adhesion materials, orthopedic device (prosthetic, bone pin, rivet, screw), and neural tube.

Most typically, the device will have an effective magnetic field and remain magnetized for a sufficient amount of time to attract an effective amount of magnetized cells to the target site to enhance a function of the tissue and/or treat a tissue injury at the target site.

Preferably, the material containing iron particles will have an effective magnetic field and remain magnetized for a sufficient amount of time to attract, capture, and/or retain an effective amount of magnetized cells to the target site for 1, 2, 3, 4, 5, 6, 7, or more days, weeks, or months, most preferably 60 days.

The materials utilizing the Fe/Pt particles are particularly suitable to enhancing repair of injury to cardiac and vascular tissue and other tissues exposed to forces and stresses caused by biologic fluid flow. Therefore, in preferred embodiments, the material will have an effective magnetic field and remain magnetized for a sufficient amount of time to attract, capture, and/or an retain an effective amount of magnetized cells to the target site to enhance repair of tissue injury, for example, vascular or cardiac injury. In some embodiments, the material will have an effective magnetic field and remain magnetized for a sufficient amount of time to attract, capture, and/or retain an effective amount of magnetized cells to the target site under a fluid flow rate of at least 1 ml/min, 5 ml/min, 10 ml/min, 25 ml/min, 50 ml/min, 75 ml/min, 100 ml/min, 150 ml/min, 250 ml/min, 500 ml/min, 750 ml/min, 1,000 ml/min.

### Myocardial Infarction

Coronary artery atherosclerosis disease (CAD) is the leading cause of death and disability in Western society and is expected to be the number one cause of death worldwide by 2020 ((Topol, et al., 2006 Hum, Mol. Genet., 15 Spec No 2:R117-23). It affects more than 11 million people in the United States alone. Occlusion of coronary vessels results in reduced blood flow to heart muscle, damage to this tissue and ultimately myocardial infarction (MI), of which there are over 1.5 million in the United States each year. MI typically causes an acute loss of myocardial tissue and an abrupt increase in loading conditions which induces left ventricular (LV) remodeling. The early phase of LV remodeling involves expansion of the infarct zone, which often results in early ventricular rupture or aneurysm formation. Late remodeling encompasses the entire LV and involves time-dependent dilatation, recruitment of border zone myocardium into the scar, distortion of ventricular shape and mural hypertrophy.

Attempts to implant living cells in damaged myocardium have met some success repairing the damaged tissue via promoting tissue regeneration (Etzion et al., J. Mol. Cell Cardiol. 33:1321-1330, 2000; Leor et al., Expert Opin. Biol. Ther. 3:1023-39, 2003; and Beltrami et al., Cell; 114:763-776, 2003). 3-D biomaterial scaffolds aimed at supporting implantation of donor cells (e.g., cardiac cells or stem cells) in the myocardium have been proposed. 3-D biomaterial scaffolds made of polysaccharide gel were successfully implanted onto damaged myocardium with promising results (Leor et al., Circulation 102:56-61, 2000). However, clinical use of such cell seeded 3-D biomaterial scaffolds is limited due to scarcity of suitable donor cells and the high risk involved in major surgery.

MI is associated with excessive and continuous damage to the extracellular matrix. It has been shown that that injection of in situ-forming alginate hydrogel into infarcts provides a temporary scaffold which attenuates adverse cardiac remodeling and dysfunction (see, for example, Leor, Journal of the American College of Cardiology, 54(11):1014-1023 (2009), and U.S. Published Application Nos. 2013/0272969, 2007/0014772).

Accordingly, the disclosed materials and methods can be employed to treat MI and/or reduce or prevent pathologies associated therewith. Compositions and methods for using injectable alginate are discussed in U.S. Published Application No. 2013/0272969. The alginate compositions discussed in U.S. Published Application No. 2013/0272969 can be modified as discussed herein to functionalize the polyalginate with magnetic particles. Although discussed here with respect to alginate, it will be appreciated that other hydrogels discussed above can be substituted to alginate in the disclosed methods. Preferred substitutable gels include, but are not limited to, those made from biocompatible polymers such as chitosan, gellan gum, carageenan, polyphosphazines, and polyacrylates,

In one embodiment, the gel is composed of alginate. For example, the functionalized alginate can have a monomer ratio between α-L-guluronic acid and β-D-mannuronic preferably ranging between 1:1 to 3:1, more preferably between 1.5:1 and 2.5:1, most preferably about 2.

The functionalized alginate can have a molecular weight ranging preferably between 1 to 300 kDa, more preferably between 5 to 200 kDa, more preferably between 10 to 100 kDa, most preferably between 20 to 50 kDa.

Crosslinking can be carried out via multivalent cations such as, but not limited to, calcium, strontium, barium, magnesium or aluminum, as well as di-, tri- and tetra-functional organic cations. In addition, polyions can be used such as, for example, poly(amino acids), poly(ethyleneimine), poly(vinylamine), poly(allylamine) and cationic polysaccharides. Most preferably, the cross linking is effected via calcium cations.

The multivalent cation salt is preferably a pharmacologically acceptable calcium salt such as, for example, calcium gluconate, calcium citrate, calcium acetate, calcium fluoride, calcium phosphate, calcium tartrate, calcium sulfate, calcium borate or calcium chloride. Most preferably, the multivalent cation salt is calcium D-gluconate (calcium gluconate).

The polymer salt is preferably a pharmacologically acceptable alginate salt such sodium, potassium, lithium, rubidium and cesium salts of alginic acid, as well as the ammonium salt, and the soluble alginates of an organic base such as mono-, di-, or tri-ethanolamine alginates, and aniline alginates. Most preferably, the polymer salt is sodium alginate.

An aqueous solution containing calcium gluconate and sodium alginate at a predetermined ratio can be prepared by combining a sodium alginate stock solution with a calcium gluconate stock solution.

The weight ratio between calcium gluconate and sodium alginate in the aqueous solution preferably ranges between 2:1 and 1:10, more preferably between 1:1 and 1:6, more preferably between 1:2 and 1:5, most preferably between 1:3 and 1:4.

The aqueous cross-linked polymer solution can be obtained by uniformly cross linking the magnetic particle-functionalized alginate via the calcium cations being present in the aqueous solution. The cross linking can be effected by (i) providing an aqueous solution containing a predetermined multivalent cation salt to polymer salt ratio and (ii) mixing the aqueous solution under conditions suitable for uniformly cross linking the polymer with the multivalent cation and yet maintaining the aqueous solution as an aqueous cross-linked polymer solution. Uniform cross linking can be effected by using a scaffold (e.g. homogenizer) capable of rigorously mixing the solution without substantially shearing the cross-linked polymer.

The final concentration (w/v) of alginate in the composition preferably ranges between 0.1 to 4%, more preferably between 0.5 and 2%, most preferably between 0.8 and 1.5%.

The final concentration (w/v) of calcium cations in the composition is preferably ranges between 0.005 and 0.1%, more preferably between 0.01 and 0.05%, more preferably between 0.02 and 0.04%, most preferably between 0.025 and 0.035%.

The composition preferably exhibits an elastic response which is equal to or greater than its viscous response under small deformation oscillatory frequencies in the linear viscoelastic limit and a shear thinning behavior in a power-law relationship.

In some embodiments, the magnetic particle-conjugated alginate composition is capable of maintaining a liquid state in storage at room temperature (e.g., at about 24-25°C) for at least 24 hr., or at least 48 hr, or at least seven days. In some embodiments, when refrigerated (e.g., at about 4-8°C) the solution is capable of maintaining a liquid state for a period of at least one month. Therefore, the composition can include a network of a viscoelastic composition which is stably maintained in a solution state. As a solution, the composition can be administered into a body tissue via a surgical needle, for example, an 18-27 gauge bore needle. The composition can be delivered into a damaged myocardium via intracoronary administration, intraventricular administration, etc.

In preferred embodiments, the composition is capable of flowing within a blood vessel, crossing out of blood capillaries and spreading into the extracellular matrix of the surrounding tissue. Following deposition within a body tissue, the composition assumes a gel state. The transition from a liquid to gel state results from the diffusion of water from the viscoelastic matrix into the surrounding extracellular medium. Once gelatinized, the viscoelastic material provides substantial mechanical support and elasticity to the body tissue, as well as scaffolding for new tissue regeneration. The alginate can be magnetized before or after implantation, but is preferably magnetized before implantation.

An effective amount of magnetized cells are administered to the subject to enhance repair of injury caused by the MI. The cells can be administered to the subject separately from the alginate, during administration of the alginate, and/or cell can be seeded into/onto the alginate *ex vivo* prior to implantation. Preferably the cells are magnetic or magnetically attractable cardiomycetes, myoblasts, fibroblasts, chondrocytes, muscle cells, smooth muscle cells, endothelial cells, mesenchymal cells or embryonic stem cells. The magnetized alginate attracts the cells to and/or retains the cells at the site of injury.

The progress of the repair can be monitored *in vivo* over time and the subject can be administered cells one or more additional times if needed. Accordingly, in some embodiments, cells are administered on two or more occasions. In some embodiments, the cells are administered according to a regular dosage regimen wherein successive rounds of cells are administered one, two, three, four, five, six, seven, or more days, weeks, months, or years apart.

In some embodiments, the materials and/or the cells or both are labeled to enhance *in vivo* imaging.

In some embodiments, the method includes administration of therapeutic, magnetic particles, and/or one or more other conventional treatments for MI, including, but not limited to, growth factors (e.g., basic fibroblast growth factor; bFGF), vascular endothelial growth factor (VEGF), insulin-like growth factor (IGF), members of the TGF-family, bone morphogenic proteins (BMP), platelet. derived growth factors, angiopoietins, and other factors such as myogenic factors, transcription factors, cytokines, and homeobox gene products, polynucleotides, polypeptides, hormones, anti-inflammatory drugs, anti-apoptotic drugs or antibiotic drugs. In some embodiments, the alginate is functionalized or loaded with one or more conventional therapeutic agents.

The present invention will be further understood by reference to the following non-limiting examples.

### Examples

### Example 1: Magnetized Cells are Retained on Magnetized Stents In Vitro

### Materials and Methods

### Synthesis of Iron/Platinum nanoparticles

The synthesis involves simultaneous chemical reduction of Pt(acac)₂ and Fe(acac)₃ by 1,2-hexadecanediol at high temperature (250 °C) in solution phase. The synthesis was handled under standard airless techniques in an argon atmosphere. The reagents were obtained from commercial sources and used without further purification. A mixture of 0.5 mmol of Pt(acac)₂, 1.0 mmol of Fe(acac)₃, and 1,2-hexadecanediol (5.0 mmol) was added to a 125 mL European flask containing a PTFE coated magnetic stir bar. Dioctyl ether (30 mL) was then transferred into the flask and the contents stirred while purging with Ar for 20 min at room temperature. The flask was then heated to 100°C and held at 100°C for 20 min. During this hold, 0.05 mmol (0.17 mL) of oleylamine and 0.05 mmol (0.16 mL) of oleic acid were injected into the flask while continuing the Ar purge. After the 20 min hold, the mixture was maintained under an Ar blanket and heated to 250°C at a rate of approximately 7°C per minute (reflux). The flask was maintained the temperature for 30 min before cooling down to room temperature under the Ar blanket. Afterward, all handling was performed open to the atmosphere.

For purification, 5 mL of the dispersion taken from the flask was added to 20 mL of ethyl alcohol (EtOH) and the mixture centrifuged (3400 rpm for 15 min). The supernatant was discarded and the precipitate redispersed in 10 mL of hexane and 5 mL of EtOH. Additional small amount of oleylamine and oleic acid might be added to aid in redispersing the nanoparticles. This dispersion was centrifuged for 15 min at 3400 rpm. The supernatant was transferred to a new centrifuge tube, discarding any precipitate that separated. An additional 15 mL of EtOH was added to this dispersion and centrifuged again. The supernatant was discarded and the remaining dark brown precipitate redispersed in hexane or dried for storage.

The FePt nanoparticles were coated with SiO₂ by base-catalyzed silica formation from tetraethylorthosilicate in a water-in-oil microemulsion in order to reduce the thermal aggregation of FePt particles during annealing at high temperature. Igepal CO-520 (8 mL) was mixed with 170 mL of cyclohexane in a 250 mL Erlenmeyer flask and stirred. FePt nanoparticles were dispersed in cyclohexane at a concentration of 1 mg/mL and then injected into the cyclohexane/Igepal solution. Approximately 1.3 mL of 30% NH₄OH aqueous solution was then added dropwise and stirred for 2-3 min, followed by the addition of 1.5 mL of tetraethylorthosilicate (TEOS). The mixture was stirred for 72 h before adding methanol to collect particles. The particles were precipitated with excess hexane and collected by centrifugation. The particles were redispersed in ethanol. The FePt@SiO₂ were "washed" using this procedure at least three times to remove excess surfactant.

The FePt@SiO₂ particles were annealed in a tube furnace. The particles were drop-cast onto a Si wafer, positioned into a 1 in. in diameter quartz tube, and then placed in the tube furnace. Annealing was performed by purging the tube and the sample for 30 min with 7% H₂/93% N₂ flow at 700 °C. Samples annealed in air were not purged. The samples were annealed at the reported temperatures for 1h. After annealing, SiO₂ coating was removed by treating the particles with 1% HF solution for 5 min.

Figure 1 is a diagram illustrating the exemplified general method of making Fe/Pt nanoparticles.

Figure 2A is a plot illustrating the size distribution of the Fe/Pt particles. Figure 2B is a hysteresis loop plot showing the magnetic moment (EMU/g) verse the external magnetic field of ((a) Fe/Pt particles annealed in N₂ at 600 °C for 30 min, and (b) Fe/Pt particles made in forming gas (N₂ 93% and H₂ 7%) at 600 °C for 30 min.

### Dye release

Coating stability of polymer layer in the presence of Fe/Pt particles, iodinated dendrimer (ID) particles or both was studied by measuring dye release. Rhodamine B (1 wt. %) was dissolved in the PLA chloroform solution and the particles were added to the solution. The solutions with or without particles were dropped on the cover glasses and dried overnight. The cover glasses were incubated in PBS at 37 °C and PBS (1 mL) was taken to measure released dye at the desired time points.

### Body clearance

PLGA particles encapsulating dir dye ((1,1'-Dioctadecyl-3,3,3',3'-Tetramethylindotricarbocyanine Iodide), Life Technologies) and/or FePt were prepared with a typical emulsion method. Briefly, PLGA (75 mg), FePt (25 mg), and dir dye (1 mg) were dissolved in chloroform, and then added drop-wise to 5% polyvinyl alcohol (PVA). The mixture was sonicated three times and then added to 0.2% PVA solution. The solvent was evaporated for 2 h under stirring and the PLGA particles were centrifuged before lyophilization. PLGA particles (48 mg/kg) containing 12 mg of FePt in PBS (250 µL) were intraperitoneally administered in mice and scanned by Bruker after 8 h, 1 d, 2 d, 4 d, 7 d, and 10 d. The mice were sacrificed to sample the blood and the organs, and measured the fluorescence (ex 740nm, em 790nm).

### Toxicology

Acute toxicity studies were performed in 10-week-old C57BL/6 female mice. Mice were dosed with indicated treatment groups on day 0. Serum concentrations of ALKP, ALT, tBIL, and BUN were measured using reagents from Teco Diagnostics at day 1, 7, and 14. C57BL/6 mice received 3 different doses of particles and compared with a PBS group. Serum clinical chemistries were within normal physiological range for alkaline phosphatase (62-209 IU/L), alanine transferase (28-152 IU/L), total bilirubin (0.1-0.9 mg/dL), and blood urea nitrogen (18-29 mg/dL). No liver or renal toxicity was observed. Mouse physiological reference ranges are from IDEXX VetTest Operator's Reference Manual (2007). Error bars represent the standard deviation. The sample size is n = 5 mice per group. Body weight was normal. EDTA anti- coagulated blood was analyzed for hematoxicity. All CBC measurements were within the normal reference range for white blood cells (1.8 - 10.7 K/µL), platelets (592 - 2971 K/µL), and hemoglobin (11.0 - 15.1 g/dL). Mouse CBC reference ranges are from Drew Scientific Hemavet 950 Reference Ranges (2010). Error bars represent the standard deviation. The sample size is n = 5 mice per group.

To ascertain levels of acute cytokines that may be induced as a result of treatment, TNF-α, IFN-γ, and IL-4 of bone marrow derived macrophages (BMM) was measured 3 days after 24 h particle treatment as a function of particle concentration. IL-4 was measured as a proxy for potential allergic responses, and TNF-α and IFN-γ for inflammatory responses. The particle groups were compared to the PBS group (negative control) as well as lipopolysaccharides (LPS) group (positive control) in the cytokine levels. No statistically significant increases in TNF-α and IL-4 were detected, and only highest dose particle (1 mg/mL) induced more IFN-γ.

The magnetic stents were placed in a bioreactor (a device for analyzing the dynamics of the attraction and capture of magnetized cells onto a magnetized stent under physiological flow conditions comparable to those observed *in vivo*) in series with comparable non-magnetic stents for evaluation of the cell capture efficiency depending on the circulation cycles, surface density of Fe/Pt particles, flow rate, number of injected magnetite cells. The magnetite and fluorescent cells (human umbilical vein endothelial cells (HUVECs)) were prepared by incorporating PLGA particles encapsulated with SPIO particles and a fluorophore. The PLGA particles were prepared by a single emulsion method and surface-stabilized with poly(vinyl alcohol) (PVA). The particles were incubated with the cells for 1 h at 37 °C and washed out with a fresh PBS. The cells with magnetites were magnetically separated and used for the cell capture studies.. Other magnetite and fluorescent cells (human derived CD34+ stem cells) were prepared by taking commercially procured CD34+ stem cells and labeling the cells by insertion of fluorescent probes (Cell TraceÒ CFSE dye) which binds to the free amines on the surface and inside of the cells. This was followed by anti-CD34+ mediated tagging with iron oxide nano and/or microparticles with subsequent washing and collection of the cells.

### Results

An *in vitro* system was developed to test the ability of magnetized stents to retain magnetized cells under simulated vascular flow conditions.

The stents used for the study was Mg100 (100 µm magnesium strut thickness 40 µm PLLA coating) in the *in vitro* system in which magnetized cells are flowed across the stent in various numbers and at various flow rates. Retention of the cells on the stent was measured in short term assays (minutes) and long term assays (hours/days).

The stent became a permanent magnet when coated with Fe/Pt particles and magnetized in 4.7 T clinical MRI scanner. SPIO particles (0.783 ± 0.135 mg/million cells) were incorporated in the cells and no significant cytotoxicity was observed. When the HUVEC cells were sequentially passed through non-magnetic stents and then magnetic stents in the bioreactor, the magnetite cells were selectively captured mainly on the magnetic stent and not much on the control stents. At the flow speed of 50 mL/min (normal physiological blood flow in proximal coronary artery), more than 47,000 cells were attracted per mm² and 10 % of the cells were captured in the first circulation. The cells were captured more efficiently (4 fold) and rapidly (10 times) when the flow rate was reduced to 25 mL/min. A lower amount of Fe/Pt particles applied on the stent recruited fewer cells.

Under conditions as described above, magnetized (FePt-PLA) and control (non-magnetized PLA only) stents were exposed according to the parameters in Table X, as follows:

**Table X. Testing parameters for retention of magnetized CD34+ stem cells**

| **Cell Count** | **Stasis Time** | **Number of Injections*** |
|---|---|---|
| 1 × 10⁵ | 1 min | 3 |
| 3.3 × 10⁵ | 3 min | 3 |
| 1 × 10⁶ | 10 min of continuous flow | |

| | | |
|---|---|---|
| ^{∗}denotes three start/stop cycles | | |

Under the parameters tested, magnetized CD34+ cells were captured by the magnetized stents at up to ~2000 cells/mm² after three injections of 3.3 × 10⁵ cells, as shown in the data in Figure 5A. By comparison, PLA-only stents captured much fewer cells (<< 500) per mm², as shown in the data in Figure 5B.

### Example 2: Encapsulation of Magnetized Cells in PLGA Particles

Cells (such as endothelial cells, macrophages or progenitor cells) can be made magnetically susceptible by intracellular incorporation of iron oxide or attachment to the surface.

### Materials and Methods

To facilitate enhanced loading of iron-oxide in cells. PLGA particles are fabricated by the double emulsion method encapsulating a high concentration of iron oxide and a dye (Coumarin 6). PLGA particles encapsulating hydrophobic superparamagnetic iron oxide (SPIO) were prepared and surface-functionalized with avidin-palmitic acid. Briefly, PLGA (107 mg) and hydrophobic SPIO (26 mg) were dissolved in chloroform (2 mL) and then added drop-wise to a vortexing solution of 5% PVA (4 mL) and the resulting mixture was sonicated three times for 10 s at an amplitude of 38% (400 W). The mixture was then added drop-wise to 100 mL of 0.2% PVA and left stirring for 3h to evaporate the solvent. Particles were collected by centrifugation at 12,000 RPM for 10 min at 4 °C and then washed three times with de-ionized water. The particles were lyophilized and stored at -20 °C until use. Particles functionalized on the surface with avidin were prepared in identical fashion with avidin-palmitate incorporated into the 5% PVA solution. Particles encapsulating Coumarin-6 and functionalized with avidin were manufactured using a modified double emulsion variation of the water-oil-water technique.

Figures 3A-3C exemplify the method of adding iron oxide particles to cells in culture (3A), which are taken up by the cells by phagocytosis or bound by a ligand such as an antibody (3B), and then isolated using an external magnet (3C).

Macrophages or endothelial cells (10⁵) cells per ml were incubated with 100 µg of PLGA particles encapsulating SPIO for 1 hr at 37C. Cells were then washed and tested for magnetic susceptibility using a 0.5 in. Neodynimum magnet.

The magnetic stents were fabricated by spraying a solution of poly(L-lactic acid) (PLLA) and Fe/Pt particles on Mg stents and then magnetized in a 4 T magnet for 24 hours. The magnetite cells (macrophages or HUVECs) were prepared by incorporating superparamagnetic iron oxide (SPIO) particles and labeled with a fluorophore. SPIO particles (0.783 ± 0.135 mg/million cells) were incorporated in the cells and no cytotoxicity was observed at this concentration. The magnetic stents were placed in a media circulating system and compared with non-magnetic stents with regard to the cell capture capability depending on: surface density of Fe/Pt particles, flow rate, number of injected magnetite cells.

### Results

The stent became a permanent magnet when coated with Fe/Pt particles, iron labeled cells were selectively captured mainly on the magnetic stent when the cells were sequentially passed through non-magnetic stents and then magnetic stents in the flow system. At the flow speed of 50 mL/min (blood flow in coronary artery), more than 47000 cells were attracted per mm² and 10 % of the cells were captured in the first circulation. The cells were captured much efficiently (4 fold) and rapidly (10 times) when the flow rate was as slow as 25 mL/min. A lower amount of Fe/Pt particles applied on the stent captured less cells.

Long term (2-72 hr) testing of the impact of Fe/Pt conc. on cell capture was conducted. The same experiment was conducted except circulation of cells was continued for 3 days (72 hours). The amount of cells captured on the stent was quantitated by fluorescence microscopy given that the labeled cells were fluorescently labeled. The number of cells captured per square area on the stent surface was ascertained using a standard relating fluorescence levels to cell number.

Migration of cells towards the magnet indicated a susceptibility to small magnetic fields in the range (0.02 T to 0.05 T).

### Example 3: Alginate Hydrogel having Fe/Pt Particles Therein

### Materials and Methods

The Fe/Pt nanoparticles discussed above can be tethered to the alginate backbone as shown in Figures 4A-4C.

A calcium sensitive magnetizable imageable hydrogel was fabricated using the CT/SPECT probe as reported in Crisone et. al. Bioconjug Chem. 2011 Sep 21;22(9):1784-9. The probe was different in that an Fe/PT construct is used with iodine and chelator with pendant functional amines for conjugation to the alginate hydrogel backbone.

This is shown in Figure 4A; Figure 4B shows functionalization of the alginate backbone with the probe and schematic of aggregation upon calcium exposure.

The *in situ*-forming alginate hydrogel was injected into myocardial infarcts to provide a temporary scaffold which attenuates adverse cardiac remodeling and dysfunction.

### Results

An absorbable biomaterial composed of calcium-crosslinked alginate solution, which displays low viscosity and, after injection into the infarct, undergoes phase transition into hydrogel was developed.

Aggregation of the probe in 1X PBS leads to an alginate (15 µg/ml) increase in the X-ray (HU) signal at 100 mg/ml of Ca²⁺, as shown in Figure 4C.

MI is associated with excessive and continuous damage to the extracellular matrix. Serial histology studies showed *in situ* formation of alginate hydrogel implant, occupied up to 50% of the scar area. The biomaterial was replaced by connective tissue within 6 weeks. Serial echocardiography studies before and 60 days after injection showed that injection of alginate biomaterial into recent (7 days) infarct increased scar thickness and attenuated left ventricular systolic and diastolic dilatation and dysfunction.

## Claims

1. A material selected from the group consisting of a hydrogel, polymeric implant, bone cement or tissue engineering scaffold comprising annealed magnetizable iron-platinum particles
wherein the annealed magnetizable iron-platinum particles are ferromagnetic particles comprising iron (Fe) and platinum (Pt) complexes having an L1₀ interior crystalline phase.

2. The material of claim 1 wherein the annealed magnetizable iron-platinum particles are magnetized.

3. The material of claim 1 wherein the annealed magnetizable iron-platinum particles are formed by annealing of iron-platinum particles at a temperature over 400°C.

4. The material of claim 3 wherein the iron-platinum of the iron-platinum particles is stabilized prior to the annealing by application of a colloidal coating of thermally resistant inorganic materials selected from the group consisting of silica, alumina powder, ceramics, iron oxides, titanium oxides, urethanes and epoxies.

5. The material of any one of claims 1-4 in a hydrogel matrix.

6. The material of any one of claims 1-4, wherein the particles are bound to an implant, prosthetic, heart valve, pacemaker leads, facial or skull reconstruction plate, tissue engineering scaffold, breast or bladder reconstruction mesh.

7. The material of any one of claims 1-4, wherein the material is a bone cement or orthopedic device such as a plate, pin, rivet, screw, or prosthetic.

8. The material of any one of claims 1-7 wherein the magnetizable iron-platinum particles are dispersed within the material, optionally, wherein the particles are dispersed in a polyester, preferably a polyhydroxy acid polymer selected from the group consisting of poly(lactic acid) (PLA), poly(glycolic acid) (PGA), poly(lactide-co-glycolide) (PLGA), or poly-Llactide (PLLA).

9. The material of any one of claims 1-8 wherein the magnetizable iron-platinum particles are dispersed in a polymer and the material comprises between 1% and 30% of the polymer by weight, inclusive, for Fe/Pt particles of greater than 50% Fe per Fe/Pt particle, and between 5% and 30% for Fe/Pt particles of less than 50% Fe per Fe/Pt particle of the polymer by weight.

10. The material of any one of claims 1-9 wherein the magnetizable iron-platinum particles comprise one or more linkers to bind the magnetizable iron-platinum particles to the material, a therapeutic or imaging agent, optionally wherein the particles are bound to a therapeutic, prophylactic or imaging agent.

11. The material of any one of claims 1-10 bound to or having incorporated thereon or therein cells.

12. An implantable medical scaffold comprising the material of any one of claims 1-11 wherein the magnetizable iron-platinum particles are magnetized and have an overall magnetic force in the range from 0.1 to 2.0 Tesla.

13. The scaffold of claim 12 comprising a therapeutic agent and/or an imaging agent.

14. The scaffold of any one of claims 12 or 13 having cells comprising a magnetic material incorporated therein or thereon bound to the scaffold, optionally wherein the scaffold is selected from the group consisting of cardiovascular, pacemaker leads, hearts valves, orthopedic and skull and facial repair scaffolds.

15. The scaffold of claim 14 selected from the group consisting of stents, grafts, bone screws, bone pins, bone plates, and plates for repair of skull and facial defects.

16. The scaffold of any one of claims 12-15 wherein the scaffold is formed in whole or in part of polymer, optionally where the scaffold is a hydrogel.

17. A medical scaffold according to any one of claims 12-16 for use in a method of promoting tissue growth in an individual wherein the medical scaffold is implanted into the individual and providing to the individual cells bound to the medical scaffold or incorporated therein magnetic particles.

18. A medical scaffold for use according to claim 17, wherein in use the scaffold is magnetized under conditions maintaining magnetization for at least sixty days.

19. A medical scaffold for use according to claim 17 or 18, wherein the scaffold is re-magnetized.

20. A medical scaffold for use according to claims 17 to 19, for use to provide an imaging agent bound to or to be incorporated into the cells or the scaffold.

21. A medical scaffold for use according to claim 20, wherein the scaffold or cells are imaged one or more times.

22. A medical scaffold for use according to claims 17-21, to provide cells, magnetize the cells, and then administer the cells to the individual; optionally, wherein the cells are selected from the group consisting of primary cells and established cell lines, embryonic stem cells, immune cells, stem cells and differentiated cells; optionally, wherein the cells are differentiated cells selected from the group consisting of fibroblasts, parenchymal cells, hematopoietic cells, epithelial cells, mesenchymal cells, neural cells, endothelial cells, myoblasts, chondrocytes, osteoblasts, osteoclasts, bone marrow cells, stem cells, and umbilical cord blood cells.

23. A medical scaffold for use according to claims 16-22, wherein the cells are obtained from the individual into whom the scaffold is implanted.

24. A medical scaffold for use according to claims 17 to 23, wherein the cells are magnetized with particles comprising iron oxide.

25. A material according to any one of claims 1-11 for use in a method for enhancing biocompatibility and/or integration of a material in a body.

## Patentansprüche

1. Material, ausgewählt aus der Gruppe bestehend aus einem Hydrogel, einem Polymerimplantat, einem Knochenzement oder einem Gerüst für die Gewebezüchtung, das geglühte magnetisierbare Eisen-Platin-Partikel enthält, wobei die geglühten magnetisierbaren Eisen-Platin-Partikel ferromagnetische Partikel sind, die Eisen (Fe)- und Platin (Pt)-Komplexe mit einer inneren kristallinen L1₀-Phase umfassen.

2. Material nach Anspruch 1, wobei die geglühten magnetisierbaren Eisen-Platin-Partikel magnetisiert sind.

3. Material nach Anspruch 1, wobei die geglühten magnetisierbaren Eisen-Platin-Partikel durch Glühen von Eisen-Platin-Partikeln bei einer Temperatur über 400 °C gebildet werden.

4. Material nach Anspruch 3, wobei das Eisen-Platin der Eisen-Platin-Partikel vor dem Glühen durch Aufbringen einer kolloidalen Beschichtung aus wärmebeständigen anorganischen Materialien, ausgewählt aus der Gruppe bestehend aus Siliciumdioxid, Aluminiumoxidpulver, Keramik, Eisenoxiden, Titanoxiden, Urethanen und Epoxiden, stabilisiert wird.

5. Material nach einem der Ansprüche 1-4 in einer Hydrogelmatrix.

6. Material nach einem der Ansprüche 1-4, wobei die Partikel an ein Implantat, eine Prothese, eine Herzklappe, Schrittmacherleitungen, eine Platte zur Gesichts- oder Schädelrekonstruktion, ein Gerüst für die Gewebezüchtung oder ein Netz zur Brust- oder Blasenrekonstruktion gebunden sind.

7. Material nach einem der Ansprüche 1-4, wobei das Material ein Knochenzement oder eine orthopädische Vorrichtung wie eine Platte, ein Stift, eine Niete, eine Schraube oder eine Prothese ist.

8. Material nach einem der Ansprüche 1-7, wobei die magnetisierbaren Eisen-Platin-Partikel in dem Material dispergiert sind, wobei die Partikel optional in einem Polyester, vorzugsweise einem Polyhydroxysäurepolymer, ausgewählt aus der Gruppe bestehend aus Poly(milchsäure) (PLA), Poly(glykolsäure) (PGA), Poly(lactid-co-glykolid) (PLGA) oder Polylactid (PLLA), dispergiert sind.

9. Material nach einem der Ansprüche 1-8, wobei die magnetisierbaren Eisen-Platin-Partikel in einem Polymer dispergiert sind und das Material zwischen 1 und 30 Gew.-% des Polymers, einschließlich für Fe/Pt-Partikel mit mehr als 50 % Fe pro Fe/Pt-Partikel, und zwischen 5 und 30 Gew.-% für Fe/Pt-Partikel mit weniger als 50 % Fe pro Fe/Pt-Partikel des Polymers enthält.

10. Material nach einem der Ansprüche 1 bis 9, wobei die magnetisierbaren Eisen-Platin-Partikel einen oder mehrere Linker umfassen, um die magnetisierbaren Eisen-Platin-Partikel an das Material, einen therapeutischen oder bildgebenden Wirkstoff zu binden, wobei die Partikel optional an einen therapeutischen, prophylaktischen oder bildgebenden Wirkstoff gebunden sind.

11. Material nach einem der Ansprüche 1-10, das an Zellen gebunden ist oder auf das oder in das Zellen eingebaut sind.

12. Implantierbares medizinisches Gerüst, umfassend das Material nach einem der Ansprüche 1-11, wobei die magnetisierbaren Eisen-Platin-Partikel magnetisiert sind und eine Gesamtmagnetkraft im Bereich von 0,1 bis 2,0 Tesla aufweisen.

13. Gerüst nach Anspruch 12, umfassend einen therapeutischen Wirkstoff und/oder einen bildgebenden Wirkstoff.

14. Gerüst nach einem der Ansprüche 12 oder 13 mit Zellen, die ein magnetisches Material darin oder darauf eingebettet enthalten, das an das Gerüst gebunden ist, wobei das Gerüst optional aus der Gruppe ausgewählt ist, die aus kardiovaskulären, Herzschrittmacherleitungs-, Herzklappen-, orthopädischen und Schädel- und Gesichtsreparaturgerüsten besteht.

15. Gerüst nach Anspruch 14, ausgewählt aus der Gruppe, bestehend aus Stents, Transplantaten, Knochenschrauben, Knochennägeln, Knochenplatten und Platten zur Reparatur von Schädel- und Gesichtsdefekten.

16. Gerüst nach einem der Ansprüche 12-15, wobei das Gerüst ganz oder teilweise aus einem Polymer gebildet ist, wobei das Gerüst optional ein Hydrogel ist.

17. Medizinisches Gerüst nach einem der Ansprüche 12-16 zur Verwendung in einem Verfahren zur Förderung des Gewebewachstums in einem Individuum, wobei das medizinische Gerüst in das Individuum implantiert wird und dem Individuum Zellen zur Verfügung gestellt werden, die an das medizinische Gerüst gebunden oder in die magnetische Partikel eingebettet sind.

18. Medizinisches Gerüst zur Verwendung nach Anspruch 17, wobei das Gerüst im Gebrauch unter Bedingungen magnetisiert wird, die die Magnetisierung für mindestens sechzig Tage aufrechterhalten.

19. Medizinisches Gerüst zur Verwendung nach Anspruch 17 oder 18, wobei das Gerüst erneut magnetisiert wird.

20. Medizinisches Gerüst zur Verwendung gemäß den Ansprüchen 17 bis 19, zur Verwendung, um einen bildgebenden Wirkstoff bereitzustellen, der an die Zellen oder das Gerüst gebunden ist oder in diese eingebettet werden soll.

21. Medizinisches Gerüst zur Verwendung nach Anspruch 20, wobei das Gerüst oder die Zellen ein oder mehrere Male abgebildet werden.

22. Medizinisches Gerüst zur Verwendung gemäß den Ansprüchen 17-21, um Zellen bereitzustellen, die Zellen zu magnetisieren und dann die Zellen dem Individuum zu verabreichen; wobei die Zellen optional ausgewählt sind aus der Gruppe bestehend aus primären Zellen und etablierten Zelllinien, embryonalen Stammzellen, Immunzellen, Stammzellen und differenzierten Zellen; wobei die Zellen optional differenzierte Zellen sind, die aus der Gruppe ausgewählt sind, die aus Fibroblasten, parenchymalen Zellen, hämatopoetischen Zellen, Epithelzellen, mesenchymalen Zellen, neuralen Zellen, Endothelzellen, Myoblasten, Chondrozyten, Osteoblasten, Osteoklasten, Knochenmarkzellen, Stammzellen und Nabelschnurblutzellen besteht.

23. Medizinisches Gerüst zur Verwendung gemäß den Ansprüchen 16-22, wobei die Zellen von dem Individuum gewonnen werden, in das das Gerüst implantiert wird.

24. Medizinisches Gerüst zur Verwendung gemäß den Ansprüchen 17 bis 23, wobei die Zellen mit Eisenoxid umfassenden Partikeln magnetisiert sind.

25. Material nach einem der Ansprüche 1-11 zur Verwendung in einem Verfahren zur Verbesserung der Biokompatibilität und/oder Integration eines Materials in einen Körper.

## Revendications

1. Matériau choisi dans le groupe constitué d'un hydrogel, d'un implant polymère, d'un ciment osseux ou d'un échafaudage d'ingénierie tissulaire comprenant des particules de fer-platine magnétisables recuites
dans lequel les particules de fer-platine magnétisables recuites sont des particules ferromagnétiques comprenant des complexes de fer (Fe) et de platine (Pt) ayant une phase cristalline intérieure L1₀.

2. Matériau selon la revendication 1, dans lequel les particules de fer-platine magnétisables recuites sont magnétisées.

3. Matériau selon la revendication 1, dans lequel les particules de fer-platine magnétisables recuites sont formées par recuit de particules de fer-platine à une température supérieure à 400 °C.

4. Matériau selon la revendication 3, dans lequel le fer-platine des particules de fer-platine est stabilisé avant le recuit par application d'un revêtement colloïdal de matériaux inorganiques thermiquement résistants choisis dans le groupe constitué de la silice, de la poudre d'alumine, de céramiques, d'oxydes de fer, d'oxydes de titane, d'uréthanes et d'époxydes.

5. Matériau selon l'une quelconque des revendications 1 à 4 dans une matrice d'hydrogel.

6. Matériau selon l'une quelconque des revendications 1 à 4, dans lequel les particules sont liées à un implant, à une prothèse, à une valvule cardiaque, à des conducteurs de stimulateur cardiaque, à une plaque de reconstruction faciale ou crânienne, à un échafaudage d'ingénierie tissulaire, à un treillis de reconstruction mammaire ou vésical.

7. Matériau selon l'une quelconque des revendications 1 à 4, dans lequel le matériau est un ciment osseux ou un dispositif orthopédique tel qu'une plaque, une broche, un rivet, une vis ou une prothèse.

8. Matériau selon l'une quelconque des revendications 1 à 7, dans lequel les particules de fer-platine magnétisables sont dispersées dans le matériau, éventuellement, dans lequel les particules sont dispersées dans un polyester, de préférence un polymère d'acide polyhydroxylé choisi dans le groupe constitué du poly(acide lactique) (PLA), du poly(acide glycolique) (PGA), du poly(lactide-co-glycolide) (PLGA) ou du poly-L-lactide (PLLA).

9. Matériau selon l'une quelconque des revendications 1 à 8, dans lequel les particules de fer-platine magnétisables sont dispersées dans un polymère et le matériau comprend entre 1 % et 30 % en poids du polymère, bornes comprises, pour des particules de Fe/Pt ayant plus de 50 % de Fe par particule de Fe/Pt, et entre 5 % et 30 % pour les particules Fe/Pt ayant moins de 50 % de Fe par particule de Fe/Pt en poids du polymère.

10. Matériau selon l'une quelconque des revendications 1 à 9, dans lequel les particules de fer-platine magnétisables comprennent un ou plusieurs lieurs pour lier les particules de fer-platine magnétisables au matériau, un agent thérapeutique ou d'imagerie, éventuellement dans lequel les particules sont liées à un agent thérapeutique, prophylactique ou d'imagerie.

11. Matériau selon l'une quelconque des revendications 1 à 10 lié à ou ayant des cellules incorporées sur celui-ci ou dans celui-ci.

12. Échafaudage médical implantable comprenant le matériau selon l'une quelconque des revendications 1 à 11, dans lequel les particules de fer-platine magnétisables sont magnétisées et ont une force magnétique globale dans la plage de 0,1 à 2,0 Tesla.

13. Échafaudage selon la revendication 12 comprenant un agent thérapeutique et/ou un agent d'imagerie.

14. Échafaudage selon l'une quelconque des revendications 12 ou 13 ayant des cellules comprenant un matériau magnétique incorporé dans celui-ci ou sur celui-ci lié à l'échafaudage, éventuellement dans lequel l'échafaudage est choisi dans le groupe constitué d'un dispositif cardiovasculaire, de conducteurs de stimulateurs cardiaques, de valvules cardiaques, de prothèses et d'échafaudages de réparation crânienne et faciale.

15. Échafaudage selon la revendication 14 choisi dans le groupe constitué de stents, de greffons, de vis à os, de broches à os, de plaques à os et de plaques pour la réparation des défauts crâniens et faciaux.

16. Échafaudage selon l'une quelconque des revendications 12 à 15, dans lequel l'échafaudage est formé en totalité ou en partie d'un polymère, éventuellement où l'échafaudage est un hydrogel.

17. Échafaudage médical selon l'une quelconque des revendications 12 à 16 destiné à être utilisé dans un procédé de promotion de la croissance tissulaire chez un individu, dans lequel l'échafaudage médical est implanté dans l'individu et fournit à l'individu des cellules liées à l'échafaudage médical ou des particules magnétiques incorporées dans celui-ci.

18. Échafaudage médical destiné à être utilisé selon la revendication 17, dans lequel lors de l'utilisation l'échafaudage est magnétisé dans des conditions maintenant la magnétisation pendant au moins soixante jours.

19. Échafaudage médical destiné à être utilisé selon la revendication 17 ou 18, dans lequel l'échafaudage est rémagnétisé.

20. Échafaudage médical destiné à être utilisé selon les revendications 17 à 19, destiné à être utilisé pour fournir un agent d'imagerie lié à ou à incorporer dans les cellules ou l'échafaudage.

21. Échafaudage médical destiné à être utilisé selon la revendication 20, dans lequel l'échafaudage ou les cellules sont imagés une ou plusieurs fois.

22. Échafaudage médical destiné à être utilisé selon les revendications 17 à 21, pour fournir des cellules, magnétiser les cellules, puis administrer les cellules à l'individu ; éventuellement, dans lequel les cellules sont choisies dans le groupe constitué de cellules primaires et de lignées cellulaires établies, de cellules souches embryonnaires, de cellules immunitaires, de cellules souches et de cellules différenciées ; éventuellement, dans lequel les cellules sont des cellules différenciées choisies dans le groupe constitué de fibroblastes, de cellules parenchymateuses, de cellules hématopoïétiques, de cellules épithéliales, de cellules mésenchymateuses, de cellules neurales, de cellules endothéliales, de myoblastes, de chondrocytes, d'ostéoblastes, d'ostéoclastes, de cellules de moelle osseuse, de cellules souches et de cellules sanguines du cordon ombilical.

23. Échafaudage médical destiné à être utilisé selon les revendications 16 à 22, dans lequel les cellules sont obtenues à partir de l'individu dans lequel l'échafaudage est implanté.

24. Échafaudage médical destiné à être utilisé selon les revendications 17 à 23, dans lequel les cellules sont magnétisées avec des particules comprenant de l'oxyde de fer.

25. Matériau selon l'une quelconque des revendications 1 à 11 destiné à être utilisé dans un procédé d'amélioration de la biocompatibilité et/ou de l'intégration d'un matériau dans un corps.
